# EUROPEAN PATENT APPLICATION

(11) **EP 4 501 950 A1**
(43) Date of publication of application: **05.02.2025**
(21) Application number: 23774013.9
(22) Date of filing: 24.03.2023
(51) Int. Cl.: C07K 14/47, C07K 14/725, A61K 38/00, A61P 35/00

(54) **HIGH-AFFINITY TCR FOR IDENTIFYING MAGE-A4 ANTIGEN, AND SEQUENCE AND USE THEREOF**

(30) Priority: 24.03.2022 CN 202210298377
(71) Applicant: XLifeSc, Ltd., Zhuhai, Guangdong 519031 (CN)
(72) Inventor: ZHAN, Kai, Zhuhai, Guangdong 519031 (CN); HUANG, Jinhua, Zhuhai, Guangdong 519031 (CN); ZHENG, Wenjing, Zhuhai, Guangdong 519031 (CN); YIN, Qianxia, Zhuhai, Guangdong 519031 (CN); TANG, Xianqing, Zhuhai, Guangdong 519031 (CN)
(74) Representative: Schulz Junghans Patentanwälte PartGmbB
(86) International application number: PCT/CN2023/083730
(87) International publication number: WO 2023/179768

(57) **Abstract**

Provided are a high-affinity TCR for identifying a MAGE-A4 antigen, and a sequence and a use thereof. The TCR has the characteristic of binding to a GVYDGREHTV-HLAA0201 complex. Also provided are a multivalent TCR complex, a nucleic acid molecule encoding the TCR, a vector comprising the nucleic acid molecule, a cell expressing the TCR, and a pharmaceutical composition comprising the substance, and a preparation method therefor.

## Description

### Cross-reference to Related Applications

This application claims the priority of Chinese patent application 202210298377.4, entitled "HIGH-AFFINITY TCR FOR IDENTIFYING MAGE-A4 ANTIGEN, AND SEQUENCE AND USE THEREOF" and filed on March 24, 2022, the entirety of which is incorporated herein by reference.

### Field of the Invention

The present disclosure relates to the technical field of biology, and particularly to a T cell receptor (TCR) capable of identifying a polypeptide derived from MAGE-A4 protein. The present disclosure also relates to the preparation and use of the TCR.

### Background of the Invention

Only two types of molecules can specifically identify antigens. One type is immunoglobulins or antibodies; the other type is the TCR, which exists as a glycoprotein on the cell membrane surface, in the form of a heterodimer including α chain/β chain or γ chain/δ chain. The composition of a TCR repertoire in the immune system is generated through V(D)J recombination in the thymus and positive and negative selection. In the peripheral environment, the TCR mediates the specific identification of the major histocompatibility complex-peptide complex (pMHC) by T cells, making it crucial for the cellular immune function of the immune system.

The TCR is the sole receptor for specific antigen peptides presented on the major histocompatibility complex (MHC), and these exogenous or endogenous peptides may represent the only signs of cellular abnormalities. In the immune system, the binding of antigen-specific TCRs to the pMHC triggers direct physical contact between T cells and antigen-presenting cells (APCs). Then, other cell membrane surface molecules on both T cells and APCs interact, leading to a series of subsequent cellular signaling events and other physiological responses. This allows different antigen-specific T cells to exert immune effects on their target cells.

The MHC class I and class II molecular ligands corresponding to the TCR are also proteins from the immunoglobulin superfamily, which have specificity for antigen presentation. Different individuals possess different MHCs, thereby presenting different short peptides from the same protein antigen to respective APC surfaces. Human MHC is commonly referred to as the HLA gene or the HLA complex.

The MAGE protein family has homologous regions that closely match the sequences of other MAGE proteins and contains peptides that are presented as HLA/peptide complexes in immune identification. Some MAGE gene family proteins (MAGE-A to MAGE-C) are expressed only in germ cells and cancers, while others (MAGE-D to MAGE-H) are widely expressed in normal tissues. MAGE-A4, as an endogenous tumor antigen, is generated intracellularly and then degraded into micro-molecule polypeptides, and binds to MHC molecules to form complexes that are presented on the cell surface. Studies have shown that GVYDGREHTV is a short peptide derived from MAGE-A4. The MAGE-A4 protein is expressed in various tumor types, including melanoma and other solid tumors such as gastric cancer, lung cancer, esophageal cancer, bladder cancer, and head and neck squamous cell carcinoma. GLYDGREHSV is a short peptide derived from MAGE-A8, which exhibits a high similarity to the short peptide GVYDGREHTV derived from MAGE-A4. For the treatment of the above-mentioned diseases, methods such as chemotherapy and radiotherapy may be employed, but they will damage normal cells in the body.

Therefore, the GVYDGREHTV-HLA A0201 complex provides a TCR for targeting the tumor cell marker. The TCR capable of binding to the GVYDGREHTV-HLA A0201 complex has significant therapeutic value for cancer treatment. For example, the TCR capable of targeting the tumor cell marker may be used for delivering cytotoxic agents or immunologic stimulants to the target cells, or be transformed into T cells, enabling the T cells expressing the TCR to destroy the tumor cells for administration to a patient in a therapeutic process known as adoptive immunotherapy. For the former purpose, the TCR with a high affinity is ideal so that the TCR can reside on the targeted cells for a long period of time. For the latter purpose, the TCR with moderate affinity is preferred. Accordingly, a person skilled in the art are focused on developing TCRs that may be used for targeting tumor cell markers for various purposes.

### Summary of the Invention

According to various embodiments of the present application, in a first aspect of the present application, a TCR is provided. The technical solution is a TCR containing an α chain variable domain and a β chain variable domain, where the TCR has an activity of binding to a GVYDGREHTV-HLA A0201 complex;
an amino acid sequence of the TCR α chain variable domain has at least 90% sequence identity to an amino acid sequence shown in SEQ ID NO.1, and an amino acid sequence of the TCR β chain variable domain has at least 90% sequence identity to an amino acid sequence shown in SEQ ID NO.2.
SEQ ID NO.1: 
GEDVEQSLFLSVREGDSSVINCTYTDSSSTYLYWYKQEPGAGLQLLTYIFS
NMDMKQDQRLTVLLNKKDKHLSLRIADTQTGDSAIYFCAEQSFGNEKLTFGT
GTRLTIIP.
SEQ ID NO.2:

NAGVTQTPKFRVLKTGQSMTLLCAQDMNHEYMYWYRQDPGMGLRLIH
YSVGEGTTAKGEVPDGYNVSRLKKQNFLLGLESAAPSQTSVYFCASSLGRAY
EQYFGPGTRLTVT.

In a preferred embodiment, the amino acid sequence of the TCR α chain variable domain and the amino acid sequence of the TCR β chain variable domain are not simultaneously an amino acid sequence of a wild-type TCR α chain variable domain and an amino acid sequence of the wild-type TCR β chain variable domain.

In a further preferred embodiment, the amino acid sequence of the TCR α chain variable domain is not the amino acid sequence shown in SEQ ID NO.1.

In another preferred embodiment, the TCR α chain variable domain contains an amino acid sequence having at least 91%, 92%, 93%, 94%, 95%, 96%, 97%, 98%, or 99% sequence identity to the sequence shown in SEQ ID NO.1, or 1, 2, 3, 4, 5, 6, 7, 8, 9, 10, or 11 amino acid residue insertions, deletions, substitutions, or combinations thereof relative to the sequence shown in SEQ ID NO.1.

In another preferred embodiment, the TCR β chain variable domain is an amino acid sequence having at least 91%, 92%, 93%, 94%, 95%, 96%, 97%, 98%, 99%, or 100% sequence identity to the sequence shown in SEQ ID NO.2, or 1, 2, 3, 4, 5, 6, 7, 8, 9, 10, or 11 amino acid residue insertions, deletions, substitutions, or combinations thereof relative to the sequence shown in SEQ ID NO.2.

In another preferred embodiment, the amino acid sequence of the TCR α chain variable domain has at least 95% sequence identity to the amino acid sequence shown in SEQ ID NO.1, and the amino acid sequence of the TCR β chain variable domain has at least 95% sequence identity to the amino acid sequence shown in SEQ ID NO.2.

In another preferred embodiment, the TCR further has an activity of binding to a GLYDGREHSV-HLAA0201 complex.

In another preferred embodiment, complementarity determining region 1α (CDR1α) in the TCR α chain variable domain is: DSSSTY (SEQ ID NO.37), and CDR2α is IFSNMDM (SEQ ID NO.38).

In another preferred embodiment, CDR3α of the TCR α chain variable domain is selected from AEHTKQNEKLT (SEQ ID NO.39), AWSQFGNEKLT (SEQ ID NO.40), AVSNFGNEKLT (SEQ ID NO.41), and AASEFGNEKLT (SEQ ID NO.42).

In another preferred embodiment, three CDRs of the TCR β chain variable domain are as follows:
CDR1β: MNHEY SEQ ID NO.59,
CDR2β: SVGEGT SEQ ID NO.60, and
CDR 3β: ASSLGRAYEQY SEQ ID NO.61.

In another preferred embodiment, the amino acid sequence of the TCR β chain variable domain is SEQ ID NO.2.

In another preferred embodiment, the number of amino acid mutations in the TCR α chain variable domain is 3-4, for example, may be 3 or 4.

In another preferred embodiment, amino acid mutation sites of the TCR α chain variable domain are at position 3, position 4, position 5, and position 6 of CDR3α; or position 2, position 3, and position 4 of CDR3α.

In another preferred embodiment, reference sequences of the three CDRs of the TCR α chain variable domain are as follows:
CDR1α: DSSSTY SEQ ID NO.37,
CDR2α: IFSNMDM SEQ ID NO.38, and
CDR3α: AEQSFGNEKLT SEQ ID NO.62;

CDR3α contains at least one of the following mutations shown in Table 1:

**Table 1**

| Residue before mutation | Residue after mutation |
|---|---|
| E at position 2 of CDR3α | W, V, A, T, S, H, Q, N, or Y |
| Q at position 3 of CDR3α | H or S |
| S at position 4 of CDR3α | T, Q, N, E, or D |
| F at position 5 of CDR3α | K, R, or H |
| G at position 6 of CDR3α | Q |

In another preferred embodiment, the amino acid mutation in CDR3α contains a mutation in Table 2:

**Table 2**

| Residue before mutation | Residue after mutation |
|---|---|
| Q at position 3 of CDR3α | S |

In another preferred embodiment, the affinity of the TCR to the GVYDGREHTV-HLAA0201 complex is at least 5 times that of a wild-type TCR.

In another preferred embodiment, the TCR is mutated in the α chain variable domain shown in SEQ ID NO.1, and the mutation is selected from one or more groups of E91W/V/A/T/S/H/Q/N/Y, Q92H/S, S93T/Q/N/E/D, F94K/R/H, and G95Q. The amino acid residue number adopts the number shown in SEQ ID NO.1.

In another preferred embodiment, the TCR has CDRs selected from Table 3.

**Table 3**

| CDR number | α-CDR1 | α-CDR2 | α-CDR3 | β-CDR1 | β-CDR2 | β-CDR3 |
|---|---|---|---|---|---|---|
| 4 | DSSSTY | IFSNMDM | AASEFGNEKLT | MNHEY | SVGEGT | ASSLGRAYEQY |
| 2 | DSSSTY | IFSNMDM | AWSQFGNEKLT | MNHEY | SVGEGT | ASSLGRAYEQY |
| 1 | DSSSTY | IFSNMDM | AEHTKQNEKLT | MNHEY | SVGEGT | ASSLGRAYEQY |
| 3 | DSSSTY | IFSNMDM | AVSNFGNEKLT | MNHEY | SVGEGT | ASSLGRAYEQY |
| 5 | DSSSTY | IFSNMDM | AASTFGNEKLT | MNHEY | SVGEGT | ASSLGRAYEQY |
| 6 | DSSSTY | IFSNMDM | AASQFGNEKLT | MNHEY | SVGEGT | ASSLGRAYEQY |
| 7 | DSSSTY | IFSNMDM | AASQRGNEKLT | MNHEY | SVGEGT | ASSLGRAYEQY |
| 8 | DSSSTY | IFSNMDM | AVSQFGNEKLT | MNHEY | SVGEGT | ASSLGRAYEQY |
| 9 | DSSSTY | IFSNMDM | AVSDFGNEKLT | MNHEY | SVGEGT | ASSLGRAYEQY |
| 10 | DSSSTY | IFSNMDM | AVSSFGNEKLT | MNHEY | SVGEGT | ASSLGRAYEQY |
| 11 | DSSSTY | IFSNMDM | ATSTFGNEKLT | MNHEY | SVGEGT | ASSLGRAYEQY |
| 12 | DSSSTY | IFSNMDM | ATSQFGNEKLT | MNHEY | SVGEGT | ASSLGRAYEQY |
| 13 | DSSSTY | IFSNMDM | ATSDFGNEKLT | MNHEY | SVGEGT | ASSLGRAYEQY |
| 14 | DSSSTY | IFSNMDM | ASSQFGNEKLT | MNHEY | SVGEGT | ASSLGRAYEQY |
| 15 | DSSSTY | IFSNMDM | AWSSFGNEKLT | MNHEY | SVGEGT | ASSLGRAYEQY |
| 16 | DSSSTY | IFSNMDM | AHSQFGNEKLT | MNHEY | SVGEGT | ASSLGRAYEQY |
| 17 | DSSSTY | IFSNMDM | AQSQFGNEKLT | MNHEY | SVGEGT | ASSLGRAYEQY |
| 18 | DSSSTY | IFSNMDM | ANSTFGNEKLT | MNHEY | SVGEGT | ASSLGRAYEQY |
| 19 | DSSSTY | IFSNMDM | AYSQFGNEKLT | MNHEY | SVGEGT | ASSLGRAYEQY |
| 20 | DSSSTY | IFSNMDM | AEHTHQNEKLT | MNHEY | SVGEGT | ASSLGRAYEQY |

Sequences of α-CDR3 are as follows:
SEQ ID NO.39: AEHTKQNEKLT
SEQ ID NO.40: AWSQFGNEKLT
SEQ ID NO.41: AVSNFGNEKLT
SEQ ID NO.42: AASEFGNEKLT
SEQ ID NO.43: AASTFGNEKLT
SEQ ID NO.44: AASQFGNEKLT
SEQ ID NO.45: AASQRGNEKLT
SEQ ID NO.46: AVSQFGNEKLT
SEQ ID NO.47: AVSDFGNEKLT
SEQ ID NO.48: AVSSFGNEKLT
SEQ ID NO.49: ATSTFGNEKLT
SEQ ID NO.50: ATSQFGNEKLT
SEQ ID NO.51: ATSDFGNEKLT
SEQ ID NO.52: ASSQFGNEKLT
SEQ ID NO.53: AWSSFGNEKLT
SEQ ID NO.54: AHSQFGNEKLT
SEQ ID NO.55: AQSQFGNEKLT
SEQ ID NO.56: ANSTFGNEKLT
SEQ ID NO.57: AYSQFGNEKLT
SEQ ID NO.58: AEHTHQNEKLT.

In another preferred embodiment, the TCR is soluble.

In another preferred embodiment, the TCR is an αβ heterodimeric TCR, containing an α chain TRAC constant region sequence and a β chain TRBC1 or TRBC2 constant region sequence.

In another preferred embodiment, the TCR contains:
(i) all or a part of a TCR α chain except its transmembrane domain, and (ii) all or a part of a TCR β chain except its transmembrane domain, where (i) and (ii) both contain a variable domain and at least a part of a constant domain of a TCR chain.

In another preferred embodiment, the TCR contains an α chain constant region and a β chain constant region, and an artificial interchain disulfide bond is contained between the α chain constant region and the β chain constant region of the TCR.

In another preferred embodiment, cysteine residues forming the artificial interchain disulfide bond between the α chain constant region and the β chain constant region of the TCR replace one or more of the following groups of sites:
Thr48 of exon 1 of TRAC*01 and Ser57 of exon 1 of TRBC1*01 or TRBC2*01;
Thr45 of exon 1 of TRAC*01 and Ser77 of exon 1 of TRBC1*01 or TRBC2*01;
Tyr10 of exon 1 of TRAC*01 and Ser17 of exon 1 of TRBC1*01 or TRBC2*01;
Thr45 of exon 1 of TRAC*01 and Asp59 of exon 1 of TRBC1*01 or TRBC2*01;
Ser15 of exon 1 of TRAC*01 and Glu15 of exon 1 of TRBC1*01 or TRBC2*01;
Arg53 of exon 1 of TRAC*01 and Ser54 of exon 1 of TRBC1*01 or TRBC2*01;
Pro89 of exon 1 of TRAC*01 and Ala19 of exon 1 of TRBC1*01 or TRBC2*01; and
Tyr10 of exon 1 of TRAC*01 and Glu20 of exon 1 of TRBC1*01 or TRBC2*01.

In another preferred embodiment, the α chain variable domain amino acid sequence of the TCR is one of SEQ ID NO.13-32; and/or the β chain variable domain amino acid sequence of the TCR is SEQ ID NO.2.

In another preferred embodiment, the TCR is selected from the groups in Table 4.

**Table 4**

| TCR number | Sequences of α chain variable domain | Sequences of β chain variable domain |
|---|---|---|
| 4 | SEQ ID NO.16 | SEQ ID NO.2 |
| 2 | SEQ ID NO.14 | SEQ ID NO.2 |
| 1 | SEQ ID NO.13 | SEQ ID NO.2 |
| 3 | SEQ ID NO.15 | SEQ ID NO.2 |
| 5 | SEQ ID NO.17 | SEQ ID NO.2 |
| 6 | SEQ ID NO.18 | SEQ ID NO.2 |
| 7 | SEQ ID NO.19 | SEQ ID NO.2 |
| 8 | SEQ ID NO.20 | SEQ ID NO.2 |
| 9 | SEQ ID NO.21 | SEQ ID NO.2 |
| 10 | SEQ ID NO.22 | SEQ ID NO.2 |
| 11 | SEQ ID NO.23 | SEQ ID NO.2 |
| 12 | SEQ ID NO.24 | SEQ ID NO.2 |
| 13 | SEQ ID NO.25 | SEQ ID NO.2 |
| 14 | SEQ ID NO.26 | SEQ ID NO.2 |
| 15 | SEQ ID NO.27 | SEQ ID NO.2 |
| 16 | SEQ ID NO.28 | SEQ ID NO.2 |
| 17 | SEQ ID NO.29 | SEQ ID NO.2 |
| 18 | SEQ ID NO.30 | SEQ ID NO.2 |
| 19 | SEQ ID NO.31 | SEQ ID NO.2 |
| 20 | SEQ ID NO.32 | SEQ ID NO.2 |

In another preferred embodiment, the TCR is humanized.

In another preferred embodiment, the TCR is obtained through isolating or purifying.

In another preferred embodiment, the TCR is a single-chain TCR.

In another preferred embodiment, the TCR is a single-chain TCR including the α chain variable domain and the β chain variable domain; the α chain variable domain and the β chain variable domain are linked by a flexible short peptide sequence (linker).

In another preferred embodiment, the TCR contains the α chain constant region and the β chain constant region; the α chain constant region is a mouse constant region, and/or the β chain constant region is a mouse constant region.

In another preferred embodiment, the TCR contains the α chain constant region and the β chain constant region; the α chain constant region is the murine constant region, and/or the β chain constant region is the murine constant region.

In another preferred embodiment, a C-or N-terminus of the α chain of the TCR is bound with a conjugate, and/or a C-or N-terminus of the β chain of the TCR is bound with a conjugate.

Preferably, the conjugate includes any one or a combination of at least two of a detectable marker, a therapeutic agent, or a protein kinase (PK) modifying moiety.

In another preferred embodiment, the therapeutic agent that binds to the TCR is an anti-CD3 antibody linked to the C-or N-terminus of the α chain of the TCR or an anti-CD3 antibody linked to the C-or N-terminus of the β chain of the TCR.

According to various embodiments of the present application, in a second aspect of the present application, a multivalent TCR complex is provided, containing any two or at least three TCRs; at least one of the TCRs is the TCR according to the first aspect.

According to various embodiments of the present application, in a third aspect of the present application, a nucleic acid molecule is provided, containing a nucleotide sequence encoding the TCR according to the first aspect of the present application or a complementary sequence of the nucleotide sequence of the TCR according to the first aspect of the present application; or
containing a nucleic acid sequence of the multivalent TCR complex according to the second aspect of the present application or a complementary sequence of the nucleic acid sequence of the multivalent TCR complex according to the second aspect of the present application.

According to various embodiments of the present application, in a fourth aspect of the present application, a vector is provided, containing the nucleic acid molecule according to the third aspect of the present application.

According to various embodiments of the present application, in a fifth aspect of the present application, a host cell is provided, containing the vector according to the fourth aspect of the present application or having a chromosome integrated with an exogenous nucleic acid molecule according to the third aspect of the present application.

According to various embodiments of the present application, in a sixth aspect of the present application, an isolated cell is provided, expressing the TCR molecule according to the first aspect of the present application.

Preferably, the isolated cell includes any one or a combination of at least two of a T cell, an NK cell, or an NKT cell.

Most preferably, the isolated cell is the T cell.

According to various embodiments of the present application, in a seventh aspect of the present application, a pharmaceutical composition is provided, containing a pharmaceutically acceptable vector, the TCR according to the first aspect of the present application, the TCR complex according to the second aspect of the present application, or the isolated cell according to the sixth aspect of the present application.

According to various embodiments of the present application, in an eighth aspect of the present application, the use of the TCR according to the first aspect, the TCR complex according to the second aspect, the isolated cell according to the sixth aspect, or the pharmaceutical composition according to the seventh aspect in the treatment of a MAGEA4 positive tumor is provided.

According to various embodiments of the present application, the present application further provides a method of treating a disease, including administering an appropriate amount of the TCR according to the first aspect of the present application, the TCR complex according to the second aspect of the present application, the isolated cell according to the sixth aspect of the present application, or the pharmaceutical composition according to the seventh aspect of the present application to a subject in need of treatment.

Preferably, the disease is a MAGE-A4 positive tumor.

According to various embodiments of the present application, in a ninth aspect of the present application, the use of the TCR according to the first aspect of the present application, the TCR complex according to the second aspect of the present application, or the isolated cell according to the sixth aspect of the present application in the preparation of a medicine for the treatment of a tumor is provided; preferably, the disease is a MAGE-A4 positive tumor.

According to various embodiments of the present application, in a tenth aspect of the present application, a preparation method of the TCR according to the first aspect of the present application is provided, including the steps of:
(i) culturing the host cell according to the fifth aspect of the present application, thereby expressing the TCR according to the first aspect of the present application; and
(ii) obtaining the TCR through isolating or purifying.

It is to be understood that within the scope of the present application, the above-mentioned features of the present application and the features described in detail below (e.g., in the examples) may be combined with each other to form new or preferred technical solutions. Due to the text limitation, it will not be repeated here.

The numerical ranges described in the present application include not only the point values exemplified above, but also any point values between the above numerical ranges not exemplified. Due to the text limitation and for the sake of brevity, the present application does not exhaustively list the specific point values included in the range.

With respect to the related art, the present application has the following beneficial effects.
(1) The affinity and/or binding half-life of the high-affinity TCR of the present disclosure for the GVYDGREHTV-HLAA0201 complex is at least 5 times that of the wild-type TCR.
(2) The high-affinity TCR of the present disclosure can specifically bind to GVYDGREHTV-HLA A0201, and meanwhile, cells transfected with the high-affinity TCR of the present disclosure can be specifically activated.
(3) Effector cells transfected with the high-affinity TCR of the present disclosure have a strong specific killing effect.

Details of one or more embodiments of the present application are set forth in the following description, and other features, objects, and advantages of the present application will become apparent from the specification and claims thereof.

### Brief Description of the Drawings

In order to more clearly illustrate the technical solutions in the embodiments of the present disclosure, the drawings required in the description of the embodiments will be briefly introduced below. Obviously, the drawings described below are only some embodiments of the present disclosure, and a person skilled in the art may obtain other drawings according to these drawings without involving any inventive effort.
Fig. 1 shows a binding curve of a soluble reference TCR, i.e., wild-type TCR, to a GVYDGREHTV-HLAA0201 complex;
Fig. 2a and Fig. 2b show activation function experimental results of effector cells transfected with a high-affinity TCR of the present application against T2 cells loaded with short peptide GVYDGREHTV and T2 cells loaded with short peptide GLYDGREHSV;
Fig. 3a and Fig. 3b show activation function experimental results of effector cells transfected with a high-affinity TCR of the present application against tumor cell lines;
Fig. 4a and Fig. 4b show killing function lactate dehydrogenase (LDH) experimental results of effector cells transfected with a high-affinity TCR of the present application against tumor cell lines;
Fig. 5 shows IFN-γ release results of effector cells transfected with a high-affinity TCR detected by ELISPOT on human normal tissue cells; and
Fig. 6 shows experimental results of the efficacy of a high-affinity TCR of the present application in mice.

### Detailed Description of the Embodiments

Hereinafter, the technical solutions of the present application will be further described with reference to specific implementations. It should be understood by a person skilled in the art that the examples are provided merely to aid in the understanding of the present application and should not be construed as specific limitations thereof.

If specific techniques or conditions are not specified in the examples, the examples are performed according to the techniques or conditions described in the literature in the art or according to the product specification. The reagents or instruments used are conventional products that are commercially available through regular channels without specifying the manufacturer.

Through extensive and in-depth studies, the present application obtains a high-affinity TCR for identifying a GVYDGREHTV short peptide (derived from a MAGE-A4 protein). The GVYDGREHTV short peptide is presented as a peptide-HLA A0201 complex. Three CDRs of the α chain variable domain of the high-affinity TCR are as follows:
CDR1α: DSSSTY SEQ ID NO.37,
CDR2α: IF SNMDM SEQ ID NO.38, and
CDR3α: AEQSFGNEKLT SEQ ID NO.62, in which mutations occur; and/or three CDRs of the β chain variable domain of the high-affinity TCR are as follows:
   CDR1β: MNHEY SEQ ID NO.59,
   CDR2β: SVGEGT SEQ ID NO.60, and
   CDR3β: ASSLGRAYEQY SEQ ID NO.61, in which mutations occur. In addition, the affinity and/or binding half-life of the TCR for the GVYDGREHTV-HLA A0201 complex after mutation is at least 5 times that of the wild-type TCR.

Before the present application is described, it is to be understood that this application is not limited to specific methods and experimental conditions described, as such methods and conditions may vary. It is also to be understood that the terminology used herein is for the purpose of describing specific implementations only, and is not intended to be limiting. The scope of the present application is limited only by the appended claims.

Unless defined otherwise, all technical and scientific terminologies used herein have the same meaning as commonly understood by a person skilled in the art to which this application belongs.

Although any methods and materials similar or equivalent to those described herein may be used in the practice or testing of the present application, preferred methods and materials are exemplified herein.

### Terminology

### TCR

The TCR may be described using the international immunogenetics information system (IMGT). The natural αβ heterodimeric TCR has an α chain and a β chain. Broadly speaking, each chain contains a variable region, a linkage region, and a constant region. The β chain also usually contains a short hypervariable region between the variable region and the linkage region, but the hypervariable region is often considered to be a part of the linkage region. The linkage region of the TCR is determined by the unique TRAJ and TRBJ of the IMGT, and the constant region of the TCR is determined by the TRAC and TRBC of the IMGT.

Each variable region contains three CDRs, i.e., CDR1, CDR2, and CDR3, chimeric in a framework sequence. In the IMGT nomenclature, different numbers of TRAV and TRBV refer to different Vα types and Vβ types, respectively. In the IMGT, the α chain constant domain has the following symbols: TRAC*01, where "TR" represents a TCR gene; "A" represents an α chain gene; C represents a constant region; "*01" represents allele 1. The β chain constant domain has the following symbols: TRBC1*01 or TRBC2*01, where "TR" represents a TCR gene; "B" represents a β chain gene; C represents a constant region; "*01" indicates allele 1. The constant region of the α chain is uniquely determined, and in the form of the β chain, there are two possible constant region genes "C1" and "C2". Constant region gene sequences of the α chain and β chain of the TCR may be obtained by a person skilled in the art from the published IMGT database.

The α chain and β chain of the TCR are generally considered to have two "domains" each, i.e., the variable domain and the constant domain. The variable domain includes the linked variable region and linkage region. Thus, in the description and claims of the present application, "TCR α chain variable domain" refers to the linked TRAV and TRAJ regions, and likewise, "TCR β chain variable domain" refers to the linked TRBV and TRBD/TRBJ regions. Three CDRs of the TCR α chain variable domain are CDR 1α, CDR 2α, and CDR 3α. Three CDRs of the TCR β chain variable domain are CDR 1β, CDR 2β, and CDR 3β. The framework sequence of the variable domain of the TCR of the present application may be murine or humanized, preferably humanized. The constant domain of the TCR contains an intracellular portion, a transmembrane region, and an extracellular portion.

In the present application, amino acid sequences of the α and β chain variable domains of the wild-type TCR capable of binding the GVYDGREHTV-HLA A0201 complex are the amino acid sequences shown in SEQ ID NO.1 and SEQ ID NO.2, respectively. In the present application, the α chain amino acid sequence and the β chain amino acid sequence of the soluble "reference TCR" are the amino acid sequences shown in SEQ ID NO.11 and SEQ ID NO.12, respectively. In the present application, the α chain extracellular amino acid sequence and the β chain extracellular amino acid sequence of the "wild-type TCR" are the amino acid sequences shown in SEQ ID NO.33 and SEQ ID NO.34, respectively. The TCR sequence used in the present application is humanized. In the present application, the α chain amino acid sequence and the β chain amino acid sequence of the "wild-type TCR" are the amino acid sequences shown in SEQ ID NO.35 and SEQ ID NO.36, respectively. In the present application, the terminologies "polypeptide of the present application", "TCR of the present application", and "TCR of the present application" are used interchangeably.
SEQ ID NO.33: 
GEDVEQSLFLSVREGDSSVINCTYTDSSSTYLYWYKQEPGAGLQLLTYIFS
NMDMKQDQRLTVLLNKKDKHLSLRIADTQTGDSAIYFCAEQSFGNEKLTFGT
GTRLTIIPNIQNPDPAVYQLRDSKSSDKSVCLFTDFDSQTNVSQSKDSDVYITD
KTVLDMRSMDFKSNSAVAWSNKSDFACANAFNNSIIPEDTFFPSPESS.
SEQ ID NO.34: 
NAGVTQTPKFRVLKTGQSMTLLCAQDMNHEYMYWYRQDPGMGLRLIH
YSVGEGTTAKGEVPDGYNVSRLKKQNFLLGLESAAPSQTSVYFCASSLGRAY
EQYFGPGTRLTVTEDLKNVFPPEVAVFEPSEAEISHTQKATLVCLATGFYPDHV
ELSWWVNGKEVHSGVSTDPQPLKEQPALNDSRYCLSSRLRVSATFWQNPRNH
FRCQVQFYGLSENDEWTQDRAKPVTQIVSAEAWGRAD.
SEQ ID NO.35: 
GEDVEQSLFLSVREGDSSVINCTYTDSSSTYLYWYKQEPGAGLQLLTYIFS
NMDMKQDQRLTVLLNKKDKHLSLRIADTQTGDSAIYFCAEQSFGNEKLTFGT
GTRLTIIPNIQNPDPAVYQLRDSKSSDKSVCLFTDFDSQTNVSQSKDSDVYITD
KTVLDMRSMDFKSNSAVAWSNKSDFACANAFNNSIIPEDTFFPSPESSCDVKLV
EKSFETDTNLNFQNLSVIGFRILLLKVAGFNLLMTLRLWSS.
SEQ ID NO.36:

NAGVTQTPKFRVLKTGQSMTLLCAQDMNHEYMYWYRQDPGMGLRLIH
YSVGEGTTAKGEVPDGYNVSRLKKQNFLLGLESAAPSQTSVYFCASSLGRAY
EQYFGPGTRLTVTEDLKNVFPPEVAVFEPSEAEISHTQKATLVCLATGFYPDHV
ELSWWVNGKEVHSGVSTDPQPLKEQPALNDSRYCLSSRLRVSATFWQNPRNH
FRCQVQFYGLSENDEWTQDRAKPVTQIVSAEAWGRADCGFTSESYQQGVLS
ATILYEILLGKATLYAVLVSALVLMAMVKRKDSRG.

### Natural interchain disulfide bond and artificial interchain disulfide bond

A set of disulfide bonds exists between juxtamembrane region Cα and Cβ chains of the natural TCR, referred to herein as "natural interchain disulfide bonds". In the present application, an artificially introduced interchain covalent disulfide bond located at a position different from that of the natural interchain disulfide bond is referred to as the "artificial interchain disulfide bond".

For convenience of description, in the present application, the position numbers of the amino acid sequences of TRAC*01 and TRBC1*01 or TRBC2*01 are in the order from the N-terminus to the C-terminus. For example, in TRBC1*01 or TRBC2*01, the 60th amino acid in the order from the N-terminus to the C-terminus is P (proline), which may be described in the present application as Pro60 of exon 1 of TRBC1*01 or TRBC2*01, and may also be described as the amino acid at position 60 of exon 1 of TRBC1*01 or TRBC2*01. In addition, in TRBC1*01 or TRBC2*01, the 61st amino acid in the order from the N-terminus to the C-terminus is Q (glutamine), which may be described in the present application as Gln61 of exon 1 of TRBC1*01 or TRBC2*01, and may also be described as the amino acid at position 61 of exon 1 of TRBC1*01 or TRBC2*01, and so on. In the present application, the position numbers of the amino acid sequences of the variable regions TRAV and TRBV are according to the position numbers listed in the IMGT. If an amino acid in TRAV is listed at position number 46 in the IMGT, it is described in the present application as the amino acid at position 46 of TRAV, and so on. In the present application, where the sequence position numbers of other amino acids are specified, they shall be specified.

### Tumor

The terminology "tumor" is meant to include all types of cancer cell growth or oncogenic processes, metastatic tissues or malignantly transformed cells, tissues, or organs, regardless of pathological types or stages of infestation. Examples of tumors include, but are not limited to, solid tumors, soft tissue tumors, and metastatic lesions. Examples of solid tumors include: malignant tumors of different organ systems, such as sarcomas, squamous cell lung carcinomas, and cancers. For example, infected prostate, lung, breast, lymph, intestines and stomach (e.g., colon), genitourinary tract (e.g., kidney and epithelial cells), and pharynx are included. Squamous cell lung carcinomas include malignant tumors such as most colon cancer, rectal cancer, renal cell carcinoma, liver cancer, non-small cell carcinoma of the lung, small intestinal cancer, and esophageal cancer. Metastatic lesions of the aforementioned cancers may likewise be treated and prevented using the method and composition of the present application.

### Detailed description of the application

It is well known that the α chain variable domain and the β chain variable domain of the TCR each contain three CDRs, similar to CDRs of antibodies. The CDR3 interacts with antigen short peptides, and the CDR1 and CDR2 interact with HLA. Thus, the CDRs of the TCR molecule determine the interaction with the antigen short peptide-HLA complex. The α chain variable domain amino acid sequence and the β chain variable domain amino acid sequence of the wild-type TCR capable of binding to the antigen short peptide GVYDGREHTV-HLA A0201 complex (i.e., GVYDGREHTV-HLA A0201 complex) are SEQ ID NO.1 and SEQ ID NO.2, respectively, which are first discovered by the inventors. The following CDRs are included.

CDRs of the α chain variable domain are as follows:
CDR1α: DSSSTY SEQ ID NO.37,
CDR2α: IFSNMDM SEQ ID NO.38, and
CDR3α: AEQSFGNEKLT SEQ ID NO.62.

CDRs of the β chain variable domain are as follows:
CDR1β: MNHEY SEQ ID NO.59,
CDR2β: SVGEGT SEQ ID NO.60, and
CDR3β: ASSLGRAYEQY SEQ ID NO.61.

In the present application, a high-affinity TCR with an affinity to the GVYDGREHTV-HLA A0201 complex of at least 5 times of that of the wild-type TCR is obtained by performing mutation screening on the CDRs.

Further, the TCR described in the present application is an αβ heterodimeric TCR. The TCR α chain variable domain contains an amino acid sequence having at least 85%, preferably, at least 90%, more preferably, at least 92%, and most preferably, at least 94% (such as may be at least 88%, 89%, 90%, 91%, 92%, 93%, 94%, 95%, 96%, 97%, 98%, and 99%) sequence identity to the amino acid sequence shown in SEQ ID NO.1;
and/or the TCR β chain variable domain contains an amino acid sequence having at least 90%, preferably, at least 92%, and more preferably, at least 94% (such as may be at least 91%, 92%, 93%, 94%, 95%, 96%, 97%, 98%, 99%, or 100%) sequence identity to the amino acid sequence shown in SEQ ID NO.2.

Further, the TCR described in the present application is a single-chain TCR. The TCR α chain variable domain contains an amino acid sequence having at least 85%, preferably, at least 90%, more preferably, at least 92%, and most preferably, at least 94% (such as may be at least 88%, 89%, 90%, 91%, 92%, 93%, 94%, 95%, 96%, 97%, 98%, and 99%) sequence identity to the amino acid sequence shown in SEQ ID NO.3;
and/or the TCR β chain variable domain contains an amino acid sequence having at least 85%, preferably, at least 90%, more preferably, at least 92%, and most preferably, at least 94% (such as may be at least 91%, 92%, 93%, 94%, 95%, 96%, 97%, 98%, and 99%) sequence identity to the amino acid sequence shown in SEQ ID NO.4.
SEQ ID NO.3: 
GEDVEQSLSLSVREGDSVVINCTYTDSSSTYLYWYKQEPGAGLQLLTYIF
SNMDMKQDQRLTVLLNKKDKHLSLRIADVQTGDSAIYFCAEQSFGNEKLTFG
TGTRLTVTP.
SEQ ID NO.4:

NAGVTQTPKYLSVKTGQSVTLLCAQDMNHEYMYWYRQDPGQGLRLIH
YSVGEGTTAKGEVPDRYNVSRLKKQNFLLGIESVTPSDTSVYFCASSLGRAYE
QYFGPGTRLTVT.

In the present application, the three CDRs of the wild-type TCR α chain variable domain SEQ ID NO.1, i.e., CDR1, CDR2, and CDR3, are located at positions 26-31, 49-55, and 90-100 of SEQ ID NO.1, respectively. Accordingly, the amino acid residue number adopts the number shown in SEQ ID NO.1. 91E is E at position 2 of CDR3α, 92Q is Q at position 3 of CDR3α, 93S is S at position 4 of CDR3α, 94F is F at position 5 of CDR3α, and 95G is G at position 6 of CDR3α.

Specifically, specific forms of the mutations in the α chain variable domain include one or more groups of E91W/V/A/T/S/H/Q/N/Y, Q92H/S, S93T/Q/N/E/D, F94K/R/H, and G95Q.

It is to be understood that the amino acid names herein are identified by international common single English letters, and the corresponding three English letters of the amino acid names are: Ala (A), Arg (R), Asn (N), Asp (D), Cys (C), Gln (Q), Glu (E), Gly (G), His (H), Ile (I), Leu (L), Lys (K), Met (M), Phe (F), Pro (P), Ser (S), Thr (T), Trp (W), Tyr (Y), and Val (V).

In the present application, Pro60 or 60P both represent proline at position 60. In addition, the specific form of the mutation described in the present application is expressed in such a way that "Q92H/S" represents the substitution of Q at position 92 by H or by S, and so on.

According to the method for site-directed mutagenesis well known to a person skilled in the art, Thr48 of exon 1 of the α chain constant region TRAC*01 of the wild-type TCR is mutated to cysteine, and Ser57 of exon 1 of the β chain constant region TRBC1*01 or TRBC2*01 is mutated to cysteine to obtain the reference TCR. The amino acid sequences of the reference TCR are the amino acid sequences shown in SEQ ID NO.11 and SEQ ID NO.12, and the mutated cysteine residues are indicated by bold letters. The above-mentioned cysteine substitutions enable the formation of an artificial interchain disulfide bond between the constant regions of the α chain and the β chain of the reference TCR to form a more stable soluble TCR, thereby enabling a more convenient assessment of the binding affinity and/or binding half-life between the TCR and the GVYDGREHTV-HLAA0201 complex. It is to be understood that the CDRs of the TCR variable region determine the affinity between the TCR and the pMHC complex. Therefore, the above-mentioned cysteine substitutions of the TCR constant regions do not affect the binding affinity and/or binding half-life of the TCR. Therefore, in the present application, the determined binding affinity between the reference TCR and the GVYDGREHTV-HLAA0201 complex is considered to be the binding affinity between the wild-type TCR and the GVYDGREHTV-HLAA0201 complex. Likewise, if the binding affinity between the TCR of the present application and the GVYDGREHTV-HLAA0201 complex is determined to be at least 10 times the binding affinity between the reference TCR and the GVYDGREHTV-HLA A0201 complex, it is equivalent to the binding affinity between the TCR of the present application and the GVYDGREHTV-HLA A0201 complex being at least 10 times the binding affinity between the wild-type TCR and the GVYDGREHTV-HLAA0201 complex.

The binding affinity (inversely proportional to a dissociation equilibrium constant KD) and binding half-life (expressed as T1/2) may be determined by any suitable method, such as detection using surface plasmon resonance technology. It will be appreciated that doubling the affinity of the TCR will result in halving the KD. T1/2 is calculated as In2 divided by the dissociation rate (Koff). Therefore, doubling T1/2 will result in halving the Koff. Preferably, the binding affinity or binding half-life of a given TCR is detected several times, e.g., three times or more, using the same test scheme, and the results are averaged. In a preferred implementation, the affinity of the soluble TCR is detected using the surface plasmon resonance (BIAcore) method in the examples herein at a temperature of 25°C and a PH of 7.1-7.5. The method detects that the dissociation equilibrium constant KD of the reference TCR for the GVYDGREHTV-HLAA0201 complex is 1.14 E-04 M, i.e., 114.00 µM. In the present application, it is considered that the dissociation equilibrium constant KD of the wild-type TCR for the GVYDGREHTV-HLA A0201 complex is also 114.00 µM. Since doubling the affinity of the TCR will result in halving the KD, if the dissociation equilibrium constant KD of the high-affinity TCR for the GVYDGREHTV-HLAA0201 complex is detected to be 1.14 E-05, i.e., 11.40 µM, it indicates that the affinity of the high-affinity TCR for the GVYDGREHTV-HLAA0201 complex is 10 times that of the wild-type TCR for the GVYDGREHTV-HLA A0201 complex. Conversions between KD value units are well known to a person skilled in the art, i.e., 1 M = 106 µM, 1 µM = 1000nM, and 1 nM = 1000 pM. In the present application, the affinity of the TCR to the GVYDGREHTV-HLAA0201 complex is at least 5 times that of a wild-type TCR.

Mutagenesis may be performed using any suitable method, including but not limited to those according to polymerase chain reaction (PCR), cloning according to restriction enzymes, or ligation-independent cloning (LIC) methods. These methods are described in detail in many standard molecular biology textbooks. Further details of PCR mutagenesis and cloning according to restriction enzymes may be found in Sambrook and Russell, (2001) Molecular Cloning-A Laboratory Manual (3rd edition) CSHL Press. Further information on the LIC method can be found in (Rashtchian, (1995) Curr Opin Biotechnol 6 (1): 30-6).

The method for generating the TCR of the present application may be, but is not limited to, screening a TCR with high affinity to the GVYDGREHTV-HLA A0201 complex from a diverse library of phage particles displaying such TCRs, as described in (Li, et al (2005) Nature Biotech 23 (3): 349-354).

It is to be understood that genes expressing α and β chain variable domain amino acids of the wild-type TCR or genes expressing α and β chain variable domain amino acids of the slightly modified wild-type TCR may be used to prepare a template TCR. The changes required to generate the high-affinity TCR of the present application are then introduced into the DNA encoding the variable domain of the template TCR.

The high-affinity TCR of the present application contains an α chain variable domain amino acid sequence of one of SEQ ID NO.13-32; and/or the β chain variable domain amino acid sequence of the TCR is SEQ ID NO.2. The amino acid sequences of the α chain variable domain and the β chain variable domain of the heterodimeric TCR molecules described in the present application are preferably selected from Table 4.

For purposes of the present application, the TCR of the present application has at least one TCR α and/or TCR β chain variable domain. The TCR typically contains both the TCR α chain variable domain and the TCR β chain variable domain. The TCR may be an αβ heterodimer or may be in a single-chain form or any other form capable of stable existence. In adoptive immunotherapy, a full-length chain (containing cytoplasmic and transmembrane domains) of the αβ heterodimeric TCR may be transfected. The TCR of the present application may be used as a targeting agent for delivering therapeutic agents to antigen-presenting cells or binding to other molecules to prepare bifunctional polypeptides for orienting effector cells. In this case, the TCR is preferably in a soluble form.

With regard to stability, it is disclosed in the related art that the introduction of an artificial interchain disulfide bond between the α and β chain constant domains of the TCR enables soluble and stable TCR molecules to be obtained, as described in the patent document PCT/CN2015/093806. Thus, the TCR of the present application may be a TCR in which an artificial interchain disulfide bond is introduced between residues of the α and β chain constant domains thereof. Cysteine residues form the artificial interchain disulfide bond between the α and β chain constant domains of the TCR. The cysteine residues may replace other amino acid residues at appropriate sites in the natural TCR to form the artificial interchain disulfide bond.

For example, Thr48 of exon 1 of TRAC*01 is replaced, and Ser57 of exon 1 of TRBC1*01 or TRBC2*01 is replaced to form a disulfide bond. Other sites where cysteine residues are introduced to form the disulfide bonds may also be:
Thr45 of exon 1 of TRAC*01 and Ser77 of exon 1 of TRBC1*01 or TRBC2*01;
Tyr10 of exon 1 of TRAC*01 and Ser17 of exon 1 of TRBC1*01 or TRBC2*01;
Thr45 of exon 1 of TRAC*01 and Asp59 of exon 1 of TRBC1*01 or TRBC2*01;
Ser15 of exon 1 of TRAC*01 and Glu15 of exon 1 of TRBC1*01 or TRBC2*01;
Arg53 of exon 1 of TRAC*01 and Ser54 of exon 1 of TRBC1*01 or TRBC2*01;
Pro89 of exon 1 of TRAC*01 and Ala19 of exon 1 of TRBC1*01 or TRBC2*01; or
Tyr10 of exon 1 of TRAC*01 and Glu20 of exon 1 of TRBC1*01 or TRBC2*01.

That is, the cysteine residues replace any set of sites in the α and β chain constant domains described above. The purpose of deleting the natural interchain disulfide bond may be achieved by truncating a maximum of 15, a maximum of 10, a maximum of 8, or fewer amino acids at one or more C-termini of the TCR constant domains of the present application so that the cysteine residue is not included, or by mutating the cysteine residue forming the natural interchain disulfide bond to another amino acid.

As described above, the TCR of the present application may contain the artificial interchain disulfide bond introduced between residues of its α and β chain constant domains. It should be noted that the TCR of the present application may contain a TRAC constant domain sequence and a TRBC1 or TRBC2 constant domain sequence with or without the artificial disulfide bond introduced between the constant domains as described above. The TRAC constant domain sequence and the TRBC1 or TRBC2 constant domain sequence of the TCR may be linked by the natural interchain disulfide bond present in the TCR.

In addition, with regard to stability, the patent document PCT/CN2016/077680 also discloses that introducing the artificial interchain disulfide bond between the α chain variable region and the β chain constant region of the TCR can significantly improve the stability of the TCR. Thus, the high-affinity TCR of the present application may also contain an artificial interchain disulfide bond between the α chain variable region and the β chain constant region. Specifically, cysteine residues forming the artificial interchain disulfide bond between the α chain variable region and the β chain constant region of the TCR replace:
amino acid at position 46 of TRAV and amino acid at position 60 of exon 1 of TRBC1*01 or TRBC2*01;
amino acid at position 47 of TRAV and amino acid at position 61 of exon 1 of TRBC1*01 or TRBC2*01;
amino acid at position 46 of TRAV and amino acid at position 61 of exon 1 of TRBC1*01 or TRBC2*01; or
amino acid at position 47 of TRAV and amino acid at position 60 of exon 1 of TRBC1*01 or TRBC2*01.

Preferably, the TCR may contain: (i) all or a part of the TCR α chain except its transmembrane domain, and (ii) all or a part of the TCR β chain except its transmembrane domain, where (i) and (ii) both contain a variable domain and at least a part of a constant domain of a TCR chain, and the α chain and the β chain form a heterodimer.

More preferably, the TCR may contain the α chain variable domain, the β chain variable domain, and all or a part of the β chain constant domain except the transmembrane domain, but the TCR does not contain the α chain constant domain. The α chain variable domain and the β chain of the TCR form a heterodimer.

With respect to stability, on the other hand, the TCR of the present disclosure also includes a TCR having a mutation in its hydrophobic core region. The mutation in the hydrophobic core region is preferably a mutation that increases the stability of the TCR of the present disclosure, as described in the patent document with publication number WO2014/206304. The TCR may be mutated at its following variable domain hydrophobic core positions:
amino acids at positions 11, 13, 19, 21, 53, 76, 89, 91, and 94 of the (α and/or β chain) variable region, α chain J gene (TRAJ) short peptide amino acids at positions 3, 5, and 7 from the bottom, and/or β chain J gene (TRBJ) short peptide amino acids at positions 2, 4, and 6 from the bottom, where the position numbers of the amino acid sequences are according to the position numbers listed in the IMGT. A person skilled in the art is aware of the above-mentioned IMGT and may obtain the position numbers of the amino acid residues of different TCRs in the IMGT according to the database.

More specifically, the TCR in which the hydrophobic core region is mutated in the present application may be a high-stability single-chain TCR including the TCR α and β chain variable domains linked by a flexible peptide chain. The CDR of the TCR variable region determines its affinity to the short peptide-HLA complex, and mutation of the hydrophobic core makes the TCR more stable without affecting its affinity to the short peptide-HLA complex. It should be noted that the flexible peptide chain in the present application may be any peptide chain suitable for linking the TCR α and β chain variable domains. A template chain for screening the high-affinity TCR constructed in example 1 of the present application is the above-mentioned high-stability single-chain TCR containing a hydrophobic core mutation. With the more stable TCR, the affinity between the TCR and the GVYDGREHTV-HLA A0201 complex can be more conveniently evaluated.

The CDRs of the α chain variable domain and the β chain variable domain of the single-chain template TCR are identical to the CDRs of the wild-type TCR. That is, the three CDRs of the α chain variable domain are CDR 1α: DSSSTY, CDR2α: IFSNMDM, and CDR3α: AEQSFGNEKLT, and the three CDRs of the β chain variable domain are CDR 1β: MNHEY, CDR2β: SVGEGT, and CDR3β: ASSLGRAYEQY. The amino acid sequence of the single-chain template TCR is the amino acid sequence shown in SEQ ID NO.9, and the nucleotide sequence is the nucleotide sequence shown in SEQ ID NO.10. A single-chain TCR including the α chain variable domain and the β chain variable domain with high affinity to the GVYDGREHTV-HLA A0201 complex is screened.

The αβ heterodimer with high affinity to the GVYDGREHTV-HLA A0201 complex of the present application is obtained by transferring the CDRs of the α and β chain variable domains of the screened high-affinity single-chain TCR to the corresponding positions of the α chain variable domain (SEQ ID NO.1) and the β chain variable domain (SEQ ID NO.2) of the wild-type TCR.

The TCR of the present application may also be provided in the form of a multivalent complex. A multivalent TCR complex of the present application contains any two or at least three (such as two, three, four, or more) polymers formed by binging to the TCR of the present application, such as a tetramer generated using a tetrameric domain of p53, or contains complexes formed by binding a plurality of TCR of the present application to another molecule. The TCR complex of the present application may be used for tracking or targeting cells presenting particular antigens in vitro or in vivo and may also be used for generating intermediates for other multivalent TCR complexes with such application.

The TCR of the present application may be used alone or may be covalently or otherwise bound to the conjugate, preferably covalently. The conjugate includes any one or a combination of at least two of a detectable marker (for diagnostic purposes, where the TCR is used for detecting the presence of cells presenting the GVYDGREHTV-HLA A0201 complex), a therapeutic agent, a PK modifying moiety (the combination includes binding or coupling).

Detectable labels for diagnostic purposes include, but are not limited to: fluorescent or luminescent markers, radioactive markers, magnetic resonance imaging (MRI) or computed tomography (CT) contrast agents, or enzymes capable of generating detectable products.

Therapeutic agents that may be bound or coupled to the TCR of the present application include, but are not limited to:
1. radionuclides (Koppe et al., 2005, Cancer metastasis reviews 24, 539);
2. biotoxin (Chaudhary et al., 1989, Nature 339, 394; Epel et al., 2002, Cancer Immunology and Immunotherapy 51, 565);
3. cytokines such as IL-2 (Gillies et al., 1992, Proceedings of the National Academy of Sciences (PNAS) 89, 1428; Card et al., 2004, Cancer Immunology and Immunotherapy 53, 345; Halin et al., 2003, Cancer Research 63, 3202);
4. antibody Fc fragments (Mosquera et al., 2005, The Journal Of Immunology 174, 4381);
5. antibody scFv fragments (Zhu et al., 1995, International Journal of Cancer 62, 319);
6. gold nanoparticles/nanorods (Lapotko et al., 2005, Cancer letters 239, 36; Huang et al., 2006, Journal of the American Chemical Society 128, 2115);
7. viral particles (Peng et al., 2004, Gene therapy 11, 1234);
8. liposomes (Mamot et al., 2005, Cancer research 65, 11631);
9. magnetic nanoparticles;
10. prodrug-activating enzymes (such as DT-diaphorase (DTD) or biphenyl hydrolase-like protein (BPHL)); and 11. chemotherapeutic agents (such as cisplatin) or any form of nanoparticles, and the like.

Antibodies or fragments thereof that bind to the TCR of the present application include anti-T cell or NK-cell determining antibodies, such as anti-CD3, anti-CD28, or anti-CD16 antibodies, the binding of the above-mentioned antibodies or fragments to the TCR can orient the effector cells to better target the target cells. According to a preferred implementation, the TCR of the present application binds to the anti-CD3 antibody or functional fragments or variants of the anti-CD3 antibody. Specifically, a fusion molecule of the TCR of the present application with the anti-CD3 single-chain antibody includes a chain variable domain amino acid sequence selected from one of SEQ ID NO.13-32; and/or the β chain variable domain amino acid sequence of the TCR is SEQ ID NO.2.

The present application also relates to a nucleic acid molecule encoding the TCR of the present application. The nucleic acid molecule of the present application may be in the form of DNA or RNA. The DNA may be a coding chain or a non-coding chain. For example, the nucleic acid sequence encoding the TCR of the present disclosure may be the same as or a degenerate variant of the nucleic acid sequence shown in the figures of the present application. To illustrate the meaning of "degenerate variant", as used herein, "degenerate variant" refers to a nucleic acid sequence encoding a protein sequence having SEQ ID NO.3, but differing from the sequence of SEQ ID NO.5.

A full-length sequence or fragments of the nucleic acid molecule of the present application may generally be obtained by, but not limited to, PCR amplification methods, recombinant methods, or artificial synthesis methods. Currently, the DNA sequence encoding the TCR (or fragments and derivatives thereof) described in the present application has been obtained entirely by chemical synthesis. The DNA sequence may then be introduced into various existing DNA molecules (or for example, vectors) and cells known in the art.

The present application also relates to a vector containing the nucleic acid molecule described in the present application, as well as a host cell genetically engineered with the vector or coding sequence of the present application.

The present application also includes an isolated cell, particularly a T cell, expressing the TCR described in the present application. There are many methods suitable for transfecting T cells with the DNA or RNA encoding the high-affinity TCR of the present application (e.g., Robbins et al., (2008) J. Immunol. 180:6116-6131). T cells expressing the high-affinity TCR described in the present application may be used for adoptive immunotherapy. A person skilled in the art will be aware of many suitable methods for performing adoptive therapies (e.g., Rosenberg et al., (2008) Nat Rev Cancer8 (4): 299-308).

The present application also provides a pharmaceutical composition, containing a pharmaceutically acceptable vector, the TCR described in the present application, the TCR complex described in the present application, or the cell presenting the TCR described in the present application.

The present application also provides a method of treating a disease, including administering an appropriate amount of the TCR described in the present application, the TCR complex described in the present application, the cell presenting the TCR described in the present application, or the pharmaceutical composition of the present application to a subject in need of treatment.

In the art, replacement with amino acids having similar properties typically does not change the function of the protein. The addition of one or several amino acids at the C-terminus and/or the N-terminus does not usually change the structure and function of the protein. Thus, the TCR of the present application also includes a TCR where at most 5, preferably at most 3, more preferably at most 2, and most preferably 1 amino acid (especially an amino acid located outside the CDR) of the present application is replaced by an amino acid having the similar property, and then the functionality is still retained.

The present application also includes a TCR with slight modifications to the TCR of the present application. The forms of the modifications (usually without changing the primary structure) include: chemically derivative forms of the TCR of present application, such as acetylation or carboxylation. Modifications also include glycosylation, such as those TCRs that result from glycosylation modifications during the synthesis and processing of the TCR of present application or during further processing steps. This modification may be accomplished by exposing the TCR to an enzyme (such as a mammalian glycosylase or deglycosylation enzyme) that performs glycosylation. The forms of the modifications also include a sequence having phosphorylated amino acid residues (e.g., phosphotyrosine, phosphoserine, and phosphothreonine). Also included is a TCR that is modified to improve the resistance to proteolysis or to optimize the solubility property.

The TCR, TCR complex, or T cell transfected with the TCR described in the present application may be provided in the pharmaceutical composition together with the pharmaceutically acceptable vector. The TCR, the multivalent TCR complex, or the cell described in the present application are typically provided as a part of a sterile pharmaceutical composition, which typically includes the pharmaceutically acceptable vector. The pharmaceutical composition may be in any suitable form (depending on the desired method of administration to the patient). It may be provided in a unit dosage form, typically in a sealed container, and as a part of a kit. This kit (but not necessarily) includes an instruction for use. A plurality of unit dosage forms may be included.

In addition, the TCR of the present application may be used alone or with other therapeutic agents by binding or coupling (e.g., formulated in the same pharmaceutical composition).

The pharmaceutical composition may further contain a pharmaceutically acceptable vector. The terminology "pharmaceutically acceptable vector" refers to a vector for the administration of a therapeutic agent. The vectors refer to those pharmaceutical vectors: they do not induce the production of antibodies harmful to the individual receiving the pharmaceutical composition by themselves and are not excessively toxic after administration. These vectors are well known to a person skilled in the art. A thorough discussion of pharmaceutically acceptable excipients may be found in Remington's Pharmaceutical Sciences (Mack Pub. Co. N. J. 1991). Such vectors include, but are not limited to, any one or a combination of at least two of saline, buffer solution, glucose, water, glycerine, ethanol, or an adjuvant.

The pharmaceutically acceptable vector of a therapeutic composition may contain liquids, such as water, saline, glycerine, and ethanol. In addition, the vector may also contain auxiliary substances, such as wetting or emulsifying agents, and pH buffering substances.

Typically, the therapeutic compositions may be prepared as injectable agents, such as liquid solutions or suspensions, and may also be prepared as solid forms suitable for being formulated into solutions, suspensions, or liquid vectors before injection.

Once formulated, the composition of the present application may be administered by conventional routes, including (but not limited to): intraocular, intramuscular, intravenous, subcutaneous, intradermal, or topical administration, preferably parenteral including subcutaneous, intramuscular, or intravenous. The subject to be prevented or treated may be an animal, especially humans.

When the pharmaceutical composition of the present application is used for an actual treatment, various dosage forms of the pharmaceutical composition may be used depending on the usage. Preferably, injection, oral administration, and the like may be exemplified.

These pharmaceutical compositions may be formulated by mixing, diluting, or dissolving according to a conventional method, and suitable pharmaceutical additives such as excipients, disintegrating agents, binders, lubricants, diluents, buffering agents, isotonicities, preservatives, wetting agents, emulsifying agents, dispersants, stabilizers, and cosolvents are occasionally added, and the formulation process may be carried out in a conventional manner depending on the dosage forms.

The pharmaceutical composition of the present application may also be administered in the form of sustained-release agents. For example, the TCR described in the present application may be incorporated into pills or microcapsules with a sustained-release polymer vector, and then the pills or microcapsules are surgically implanted into the tissue to be treated. As examples of the sustained-release polymer, ethylene-vinyl acetate copolymer, polyhydrometaacrylate, polyacrylamide, polyvinylpyrrolidone, methyl cellulose, lactic acid polymer, lactic acid-glycolic acid copolymer, and the like may be exemplified, and preferably biodegradable polymers such as lactic acid polymer and lactic acid-glycolic acid copolymer may be exemplified.

When the pharmaceutical composition of the present application is used for an actual treatment, the TCR or TCR complex of the present application or the cell presenting the TCR of the present application as active ingredients may be rationally determined according to the body weight, age, sex, degree of symptoms of each patient to be treated, and a reasonable amount can be decided by a physician.

The main advantages of the present application are as follows.
(1) The affinity and/or binding half-life of the high-affinity TCR of the present application for the GVYDGREHTV-HLA A0201 complex is at least 5 times that of the wild-type TCR.
(2) The high-affinity TCR of the present application can specifically bind to GVYDGREHTV-HLA A0201, and meanwhile, cells transfected with the high-affinity TCR of the present application can be specifically activated.
(3) Effector cells transfected with the high-affinity TCR of the present application have a strong specific killing effect.

The following specific examples further illustrate the present application. It is to be understood that these examples are for illustrative purposes only and are not intended to limit the scope of the present application. Experimental methods without specifying the specific conditions in the following examples are generally performed according to conventional conditions, such as (Sambrook, Russell et al., Molecular Cloning-A Laboratory Manual (3rd edition) (2001) CSHL Press) or as recommended by the manufacturer. Percentages and parts are calculated by weight unless otherwise indicated.

### Materials and Methods

The experimental materials used in the examples of the present application are available from commercial sources unless otherwise specified. E. coli DH5α is purchased from Tiangen, E. coli BL21 (DE3) is purchased from Tiangen, E. coli Tuner (DE3) is purchased from Novagen, and plasmid pET28a is purchased from Novagen.

### Example 1

### Generation of stable single-chain TCR template chain with hydrophobic core mutations

In the present application, the method of site-directed mutagenesis was used, and a stable single-chain TCR molecule was constructed by linking the α and β chain variable domains of the TCR with a flexible short peptide (linker) as described in WO2014/206304. Its amino acid and DNA sequences were as shown in SEQ ID NO.9 and SEQ ID NO.10, respectively. The high-affinity TCR molecules were screened using the single-chain TCR molecule as a template. The α chain variable domain sequence of the template chain was the amino acid sequence shown in SEQ ID NO.3, and the β chain variable domain sequence was the amino acid sequence shown in SEQ ID NO.4, and their corresponding DNA sequences were the nucleotide sequences shown in SEQ ID NO.5 and SEQ ID NO.6. The amino acid sequence and the DNA sequence of the flexible short peptide (linker) were the nucleotide sequences shown in SEQ ID NO.7 and SEQ ID NO.8, respectively.
SEQ ID NO.5: 
ggtgaagatgttgaacaaagcctgagcctgagcgtgcgcgaaggcgatagcgtggttattaattgcacctataccg
atagtagcagtacctatctgtattggtataaacaggaaccgggcgcaggtctgcagctgctgacctatattttcagtaatatgg
atatgaagcaggatcagcgcctgaccgttctgctgaataagaaagataaacatctgagcctgcgtattgccgatgttcagac
cggtgacagcgcaatctatttctgtgccgaacagagttttggcaatgaaaagctgacctttggtaccggtacccgtctgaccg
ttaccccg.
SEQ ID NO.6: 
aatgccggtgttacccagaccccgaaatatctgagcgttaaaaccggccagagtgttaccctgctgtgcgcccagg
atatgaatcatgaatatatgtattggtaccgccaggaccctggccagggtctgcgcttaattcattatagtgtgggtgaaggta
ccaccgcaaaaggcgaagtgccggatcgttataatgtgagtcgcctgaagaaacagaattttctgctgggcattgaaagtgt
gaccccgagtgataccagcgtgtatttctgtgcaagtagcctgggtcgtgcctatgaacagtattttggcccgggtacccgc
ctgaccgtgacc.
SEQ ID NO.7:
   GGGSEGGGSEGGGSEGGGSEGGTG.
SEQ ID NO.8:
   ggtggcggcagtgaaggcggtggtagcgaaggcggcggcagcgaaggtggtggtagtgaaggtggtaccggc.
SEQ ID NO.9: 
GEDVEQSLSLSVREGDSVVINCTYTDSSSTYLYWYKQEPGAGLQLLTYIF
SNMDMKQDQRLTVLLNKKDKHLSLRIADVQTGDSAIYFCAEQSFGNEKLTFG
TGTRLTVTPGGGSEGGGSEGGGSEGGGSEGGTGNAGVTQTPKYLSVKTGQSV
TLLCAQDMNHEYMYWYRQDPGQGLRLIHYSVGEGTTAKGEVPDRYNVSRL
KKQNFLLGIESVTPSDTSVYFCASSLGRAYEQYFGPGTRLTVT.
SEQ ID NO.10:

ggtgaagatgttgaacaaagcctgagcctgagcgtgcgcgaaggcgatagcgtggttattaattgcacctataccg
atagtagcagtacctatctgtattggtataaacaggaaccgggcgcaggtctgcagctgctgacctatattttcagtaatatgg
atatgaagcaggatcagcgcctgaccgttctgctgaataagaaagataaacatctgagcctgcgtattgccgatgttcagac
cggtgacagcgcaatctatttctgtgccgaacagagttttggcaatgaaaagctgacctttggtaccggtacccgtctgaccg
ttaccccgggtggcggcagtgaaggcggtggtagcgaaggcggcggcagcgaaggtggtggtagtgaaggtggtacc
ggcaatgccggtgttacccagaccccgaaatatctgagcgttaaaaccggccagagtgttaccctgctgtgcgcccaggat
atgaatcatgaatatatgtattggtaccgccaggaccctggccagggtctgcgcttaattcattatagtgtgggtgaaggtacc
accgcaaaaggcgaagtgccggatcgttataatgtgagtcgcctgaagaaacagaattttctgctgggcattgaaagtgtga
ccccgagtgataccagcgtgtatttctgtgcaagtagcctgggtcgtgcctatgaacagtattttggcccgggtacccgcctg
accgtgacc.

A target gene carrying the template chain was double digested by Nco I and Not I and linked to the pET28a vector which was double digested by Nco I and Not I. The linking product was transformed into E. coli DH5α, plated on a kanamycin-containing LB plate, and cultured in an inverted position at 37°C overnight. Positive clones were picked for PCR screening, and positive recombinants were sequenced. After confirming the correct sequence, recombinant plasmids were extracted and transformed into E.coli BL21 (DE3) for expression.

### Example 2

### Expression, renaturation, and purification of stable single-chain TCR constructed in example 1

BL21 (DE3) colonies containing the recombinant plasmid pET28a-template chain prepared in example 1 were all inoculated in a kanamycin-containing LB medium and cultured at 37°C until the OD₆₀₀ was 0.6-0.8, IPTG was added to a final concentration of 0.5 mM, and further cultured at 37°C for 4 h. A cell precipitate was obtained by centrifugation at 5000 rpm for 15 min and lysed with Bugbuster Master Mix (Merck). Inclusion bodies were recovered by centrifugation at 6000 rpm for 15 min and washed with Bugbuster (Merck) to remove cell debris and membrane components. Inclusion bodies were collected by centrifugation at 6000 rpm for 15 min. The inclusion bodies were dissolved in a buffer solution (20 mM Tris-HCl pH 8.0, 8 M urea), and insolubles were removed by high-speed centrifugation. The supernatant was aliquoted after quantification by the BCA method and stored at -80°C for use.

2.5 mL of buffer solution (6 M Gua-HCl, 50 mM Tris-HCl pH 8.1, 100 mM NaCl, and 10 mM EDTA) was added to 5 mg of solubilized single-chain TCR inclusion body protein, and then DTT was added to a final concentration of 10 mM and treated at 37°C for 30 min. The above treated single-chain TCRs were added dropwise by a syringe to 125 mL of renaturation buffer solution (100 mM Tris-HCl pH 8.1, 0.4 M L-arginine, 5 M urea, 2 mM EDTA, 6.5 mM β-mercapthoethylamine, and 1.87 mM cystamine) and stirred at 4°C for 10 min, and then the renaturation solution was loaded into a cellulose membrane dialysis bag with 4 kDa cut-off. The dialysis bag was placed in 1 L of pre-cooled water and slowly stirred overnight at 4°C. After 17 hours, the dialysate was changed to 1 L of pre-cooled buffer solution (20 mM Tris-HCl pH8.0), and dialysis was continued for 8 h at 4°C. Then, the dialysate was changed to the same fresh buffer solution, and dialysis was continued overnight. After 17 hours, the sample was filtered through a 0.45 µm filter membrane and degassed under vacuum, and passed through an anion-exchange column (HiTrap Q HP, GE Healthcare). The protein was purified using a 0-1M NaCl linear gradient eluent prepared with 20 mM Tris-HCl pH8.0. Collected elution fractions were subjected to SDS-PAGE analysis. Fractions containing the single-chain TCRs were concentrated and further purified using a gel filtration column (Superdex 75 10/300, GE Healthcare). Target fractions were also subjected to SDS-PAGE analysis.

The elution fractions for BIAcore analysis were further tested for purity through a gel filtration method. The conditions were as follows. The chromatographic column was Agilent Bio SEC-3 (300 A, ϕ7.8×300 mm), the mobile phase was 150 mM phosphate buffer solution, the flow rate was 0.5 mL/min, the column temperature was 25°C, and the ultraviolet detection wavelength was 214 nm.

### Example 3

### Binding characterization

### BIAcore analysis

The binding activity of TCR molecules to the GVYDGREHTV-HLA A0201 complex was detected using a BIAcore T200 real-time analysis system. An anti-streptavidin antibody (GenScript) was added to a coupling buffer solution (10 mM sodium acetate buffer solution, pH4.77). Then, the antibody flowed over a CM5 chip previously activated with EDC and NHS so that the antibody was immobilized on the chip surface. Finally, the coupling process was completed by blocking the unreacted activated surface with a hydrochloric acid solution of ethanolamine at a coupling level of approximately 15,000 RU. The conditions were as follows. The temperature was 25°C, and the pH was 7.1-7.5.

A low concentration of streptavidin flowed over the antibody-coated chip surface, and then the GVYDGREHTV-HLA A0201 complex flowed over a detection channel. Another channel served as a reference channel. 0.05 mM biotin flowed over the chip at a flow rate of 10 pL/min for 2 min to block the remaining binding sites of streptavidin. The affinity was determined by a single-cycle kinetic analysis method. The TCRs were diluted with a HEPES-EP buffer solution (10 mM HEPES, 150 mM NaCl, 3 mM EDTA, 0.005% P20, pH7.4) to several different concentrations, and flowed over the chip surface in sequence at a flow rate of 30 µL/min. The binding time for each sample injection was 120 s. After the last sample injection, dissociation was carried out for 600 s. After each round of measurement, the chip was regenerated with 10 mM Gly-HCl at pH1.75. Kinetic parameters were calculated using BIAcore Evaluation software.

A preparation process of the above-mentioned GVYDGREHTV-HLA A0201 complex was as follows.
a. Purification: 100 mL of E. coli bacterial solution induced to express heavy chain or light chain was collected and centrifuged at 8000 g and 4°C for 10 min. Then, the bacteria were washed once with 10 mL of PBS, then resuspended with vigorous shaking using 5 ml of BugBuster Master Mix Extraction Reagents (Merck), incubated with rotation at room temperature for 20 min, and then centrifuged at 6000 g and 4°C for 15 min. The supernatant was discarded, and inclusion bodies were collected.
   The inclusion bodies were resuspended in 5 mL of BugBuster Master Mix and incubated with rotation at room temperature for 5 min. 30 mL of 10-fold diluted BugBuster was added, mixed well, and centrifuged at 6000 g and 4°C for 15 min. The supernatant was discarded. 30 mL of 10-fold diluted BugBuster was added to resuspend the inclusion bodies, mixed well, and centrifuged at 6000 g and 4°C for 15 min, repeating twice. 30 mL of 20 mM Tris-HCl pH 8.0 was added to resuspend the inclusion bodies, mixed well, and centrifuged at 6000 g and 4°C for 15 min. Finally, the inclusion bodies were dissolved with 20 mM Tris-HCl 8M urea. The purity of the inclusion bodies was detected with SDS-PAGE, and the concentration was detected with a BCA kit.
b. Renaturation: the synthetic short peptide GVYDGREHTV (Jiangsu Jinsirui Biotechnology Co., Ltd.) was dissolved in DMSO to a concentration of 20 mg/mL. The inclusion bodies of the light chain and the heavy chain were dissolved with 8 M urea, 20 mM Tris pH 8.0, and 10 mM DTT and further denatured by adding 3 M guanidine hydrochloride, 10 mM sodium acetate, and 10 mM EDTA before renaturation. GVYDGREHTV peptide was added to a renaturation buffer solution (0.4 M L-arginine, 100 mM Tris pH 8.3, 2 mM EDTA, 0.5 mM oxidized glutathione, 5 mM reduced glutathione, and 0.2 mM PMSF, cooled to 4°C) at 25 mg/L (final concentration), and then 20 mg/L of light chain and 90 mg/L of heavy chain were added successively (final concentration, heavy chain was added in three times, 8 h/time). Renaturation was performed at 4°C for at least 3 days to completion, and an SDS-PAGE detected whether renaturation could be successful.
c. Purification after renaturation: the renaturation buffer solution was replaced with 10 times the volume of 20 mM Tris pH8.0 for dialysis at least twice to reduce the ionic strength of the solution sufficiently. After dialysis, a protein solution was filtered through a 0.45 um cellulose acetate filter membrane and loaded onto a HiTrap Q HP (General Electric Company, GE) anion-exchange column (5 mL bed volume). The protein was eluted using an Akta purifier (GE) with a 0-400 mM NaCl linear gradient solution formulated with 20 mM Tris pH8.0. The pMHC was eluted at approximately 250 mM NaCl. The peak fractions were collected, and the purity was detected with SDS-PAGE.
d. Biotinylation: the purified pMHC molecules were concentrated using a Millipore ultrafiltration tube, and meanwhile, the buffer solution was replaced with 20 mM Tris pH 8.0. Then, biotinylation reagents 0.05 M Bicine pH 8.3, 10 mM ATP, 10 mM MgOAc, 50 µM D-Biotin, 100 ug/mL BirA enzyme (GST-BirA) were added. The mixture was incubated overnight at room temperature, and SDS-PAGE detected whether biotinylation was complete.
e. Purification of the biotinylated complex: the biotinylated pMHC molecules were concentrated to 1 ml using the Millipore ultrafiltration tube, and the biotinylated pMHC was purified by gel filtration chromatography using the Akta purifier (GE). A filtered PBS pre-equilibrated HiPrepTM 16/60 S200 HR column (GE) was used, and 1 mL of the concentrated biotinylated pMHC molecules was loaded and eluted with PBS at a flow rate of 1 mL/min. The biotinylated pMHC molecules eluted as a single peak at approximately 55 mL. Protein-containing fractions were combined and concentrated using the Millipore ultrafiltration tube. Protein concentration was determined by a BCA method (Thermo), and biotinylated pMHC molecules were aliquoted and stored at -80°C by adding the protease inhibitor cocktail (Roche).

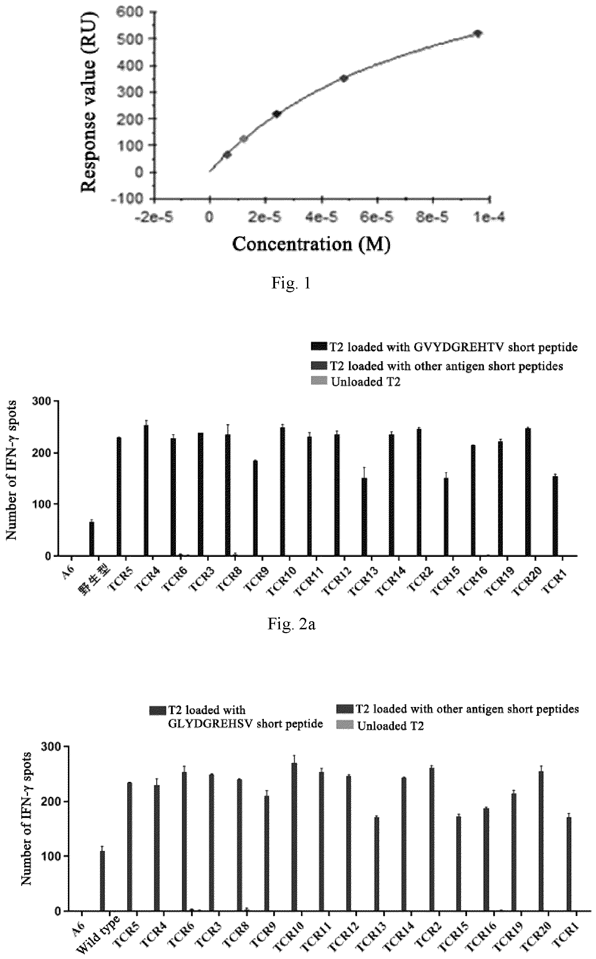

The binding curve of the soluble reference TCR, i.e., wild-type TCR, to the GVYDGREHTV-HLA A0201 complex was measured, and the results were shown in Fig. 1.

### Example 4

### Generation of high-affinity TCR

Phage display technology is one means of generating a library of high-affinity variants of the TCR to screen high-affinity variants. The TCR phage display and screening method described by Li et al., ((2005) Nature Biotech 23 (3): 349-354) was applied to the single-chain TCR template in example 1. A library of high-affinity TCRs was created by mutating the CDRs of the template chain for panning. After several rounds of panning, the phage library bound specifically to the corresponding antigens, and single clones were picked and analyzed.

Mutations in the CDRs of the screened high-affinity single-chain TCR were introduced into the corresponding sites of the variable domain of the αβ heterodimeric TCR, and the affinity to the GVYDGREHTV-HLA A0201 complex was detected by BIAcore. The introduction of the high-affinity mutation sites in the CDRs described above employed the site-directed mutagenesis method well known to a person skilled in the art. The α and β chain variable domain amino acid sequences of the wild-type TCR were the amino acid sequences shown in SEQ ID NO.1 and SEQ ID NO.2, respectively.

It should be noted that in order to obtain a more stable soluble TCR so as to more conveniently evaluate the binding affinity and/or the binding half-life between the TCR and the GVYDGREHTV-HLA A0201 complex, the αβ heterodimeric TCR could be a TCR having a cysteine residue introduced into each of the constant regions of the α and β chains to form an artificial interchain disulfide bond. In this example, the amino acid sequences of the α and β chains of the TCR after the introduction of the cysteine residue were the amino acid sequences shown in SEQ ID NO.11 and SEQ ID NO.12, respectively, and the introduced cysteine residue was shown in bold and underlined letters.
SEQ ID NO.11: 
GEDVEQSLFLSVREGDSSVINCTYTDSSSTYLYWYKQEPGAGLQLLTYIFS
NMDMKQDQRLTVLLNKKDKHLSLRIADTQTGDSAIYFCAEQSFGNEKLTFGT
GTRLTIIPNIQNPDPAVYQLRDSKSSDKSVCLFTDFDSQTNVSQSKDSDVYITD
KTVLDMRSMDFKSNSAVAWSNKSDFACANAFNNSIIPEDTFFCSPESS.
SEQ ID NO.12:

NAGVTQTPKFRVLKTGQSMTLLCAQDMNHEYMYWYRQDPGMGLRLIH
YSVGEGTTAKGEVPDGYNVSRLKKQNFLLGLESAAPSQTSVYFCASSLGRAY
EQYFGPGTRLTVTEDLKNVFPPEVAVFEPSECEISHTQKATLVCLATGFYPDHV
ELSWWVNGKEVHSGVSTDPQPLKEQPALNDSRYALSSRLRVSATFWQNPRNH
FRCQVQFYGLSENDEWTQDRAKPVTQIVSAEAWGRAD.

The extracellular sequence genes of the TCR α and β chains to be expressed were synthesized and inserted into the expression vector pET28a+ (Novagene) through the standard methodss described in Molecular Cloning a Laboratory Manual (3rd edition, Sambrook and Russell). The cloning sites upstream and downstream were Nco I and Not I, respectively. Mutations in the CDRs were introduced by overlap PCR well known to a person skilled in the art. The inserted fragments were sequenced and confirmed to be correct.

### Example 5

### Expression, renaturation, and purification of high-affinity TCR

The expression vectors of TCR α and β chains were transformed into the expression bacteria BL21 (DE3) by a chemical transformation method. The bacteria were grown in an LB culture solution and were induced with a final concentration of 0.5 mM IPTG at OD₆₀₀=0.6. The inclusion bodies formed after expression of the TCR α and β chains were extracted through BugBuster Mix (Novagene) and washed several times with a BugBuster solution. The inclusion bodies were finally dissolved in 6 M guanidine hydrochloride, 10 mM dithiothreitol (DTT), 10 mM ethylenediamine tetraacetic acid (EDTA), and 20 mM Tris (pH8.1).

The dissolved TCR α and β chains were rapidly mixed in a 1:1 mass ratio in 5 M urea, 0.4 M arginine, 20 mM Tris (pH8.1), 3.7 mM cystamine, 6.6 mM β-mercapoethylamine (4°C) to a final concentration of 60 mg/mL. After mixing, the solution was placed in 10 times the volume of deionized water for dialysis (4°C). After 12 hours, the deionized water was replaced with a buffer solution (20 mM Tris, pH8.0) and continued for dialysis at 4°C for 12 hours. The dialyzed solution was filtered through a 0.45 uM filter membrane and purified through an anion-exchange column (HiTrap Q HP, 5ml, GE Healthcare). The TCR with elution peaks containing the renatured successful α and β dimer were confirmed by SDS-PAGE gel. The TCR was then further purified by gel filtration chromatography (HiPrep 16/60, Sephacryl S-100 HR, GE Healthcare). The purity of the purified TCR was determined by SDS-PAGE to be greater than 90%, and the concentration was determined by the BCA method.

### Example 6

### BIAcore analysis results

The affinity of the αβ heterodimeric TCR introducing high-affinity CDRs to the GVYDGREHTV-HLA A0201 complex was detected using the method described in example 3.

New TCR α chain variable domain amino acid sequences obtained in the present application were the amino acid sequences shown in SEQ ID NO.13-32. Since the CDR of the TCR molecule determines its affinity to the corresponding pMHC complex, a person skilled in the art would have been able to predict that the αβ heterodimeric TCR introducing high-affinity mutation sites also has a high affinity to the GVYDGREHTV-HLA A0201 complex. The expression vector was constructed using the method described in example 4, the αβ heterodimeric TCR introducing high-affinity mutations described above was expressed, renatured, and purified using the method described in example 5, and then the affinity to the GVYDGREHTV-HLA A0201 complex was determined using BIAcore T200, as shown in Table 5.

**Table 5**

| TCR number | α chain variable domain sequences | β chain variable domain sequences | kD |
|---|---|---|---|
| 4 | SEQ ID NO.16 | SEQ ID NO.2 | 4.40E-06 |
| 2 | SEQ ID NO.14 | SEQ ID NO.2 | 3.30E-06 |
| 1 | SEQ ID NO.13 | SEQ ID NO.2 | 1.62E-05 |
| 3 | SEQ ID NO.15 | SEQ ID NO.2 | 5.83E-06 |
| 5 | SEQ ID NO.17 | SEQ ID NO.2 | 3.01E-06 |
| 6 | SEQ ID NO.18 | SEQ ID NO.2 | 2.24E-06 |
| 7 | SEQ ID NO.19 | SEQ ID NO.2 | 7.93E-07 |
| 8 | SEQ ID NO.20 | SEQ ID NO.2 | 2.25E-06 |
| 9 | SEQ ID NO.21 | SEQ ID NO.2 | 1.45E-05 |
| 10 | SEQ ID NO.22 | SEQ ID NO.2 | 8.02E-06 |
| 11 | SEQ ID NO.23 | SEQ ID NO.2 | 7.12E-06 |
| 12 | SEQ ID NO.24 | SEQ ID NO.2 | 3.31E-06 |
| 13 | SEQ ID NO.25 | SEQ ID NO.2 | 1.30E-05 |
| 14 | SEQ ID NO.26 | SEQ ID NO.2 | 3.69E-06 |
| 15 | SEQ ID NO.27 | SEQ ID NO.2 | 1.01E-05 |
| 16 | SEQ ID NO.28 | SEQ ID NO.2 | 3.13E-06 |
| 17 | SEQ ID NO.29 | SEQ ID NO.2 | 9.40E-06 |
| 18 | SEQ ID NO.30 | SEQ ID NO.2 | 4.17E-06 |
| 19 | SEQ ID NO.31 | SEQ ID NO.2 | 4.35E-06 |
| 20 | SEQ ID NO.32 | SEQ ID NO.2 | 5.21E-06 |

As can be seen from Table 5, the affinity of the heterodimeric TCR was at least 5 times that of the wild-type TCR to the GVYDGREHTV-HLAA0201 complex.
SEQ ID NO.13: 
GEDVEQSLFLSVREGDSSVINCTYTDSSSTYLYWYKQEPGAGLQLLTYIFS
NMDMKQDQRLTVLLNKKDKHLSLRIADTQTGDSAIYFCAEHTKQNEKLTFGT
GTRLTIIP.
SEQ ID NO.14 
GEDVEQSLFLSVREGDSSVINCTYTDSSSTYLYWYKQEPGAGLQLLTYIFS
NMDMKQDQRLTVLLNKKDKHLSLRIADTQTGDSAIYFCAWSQFGNEKLTFGT
GTRLTIIP.
SEQ ID NO.15 
GEDVEQSLFLSVREGDSSVINCTYTDSSSTYLYWYKQEPGAGLQLLTYIFS
NMDMKQDQRLTVLLNKKDKHLSLRIADTQTGDSAIYFCAVSNFGNEKLTFGT
GTRLTIIP.
SEQ ID NO.16 
GEDVEQSLFLSVREGDSSVINCTYTDSSSTYLYWYKQEPGAGLQLLTYIFS
NMDMKQDQRLTVLLNKKDKHLSLRIADTQTGDSAIYFCAASEFGNEKLTFGT
GTRLTIIP.
SEQ ID NO.17 
GEDVEQSLFLSVREGDSSVINCTYTDSSSTYLYWYKQEPGAGLQLLTYIFS
NMDMKQDQRLTVLLNKKDKHLSLRIADTQTGDSAIYFCAASTFGNEKLTFGT
GTRLTIIP.
SEQ ID NO.18 
GEDVEQSLFLSVREGDSSVINCTYTDSSSTYLYWYKQEPGAGLQLLTYIFS
NMDMKQDQRLTVLLNKKDKHLSLRIADTQTGDSAIYFCAASQFGNEKLTFGT
GTRLTIIP.
SEQ ID NO.19 
GEDVEQSLFLSVREGDSSVINCTYTDSSSTYLYWYKQEPGAGLQLLTYIFS
NMDMKQDQRLTVLLNKKDKHLSLRIADTQTGDSAIYFCAASQRGNEKLTFGT
GTRLTIIP.
SEQ ID NO.20 
GEDVEQSLFLSVREGDSSVINCTYTDSSSTYLYWYKQEPGAGLQLLTYIFS
NMDMKQDQRLTVLLNKKDKHLSLRIADTQTGDSAIYFCAVSQFGNEKLTFGT
GTRLTIIP.
SEQ ID NO.21 
GEDVEQSLFLSVREGDSSVINCTYTDSSSTYLYWYKQEPGAGLQLLTYIFS
NMDMKQDQRLTVLLNKKDKHLSLRIADTQTGDSAIYFCAVSDFGNEKLTFGT
GTRLTIIP.
SEQ ID NO.22 
GEDVEQSLFLSVREGDSSVINCTYTDSSSTYLYWYKQEPGAGLQLLTYIFS
NMDMKQDQRLTVLLNKKDKHLSLRIADTQTGDSAIYFCAVSSFGNEKLTFGT
GTRLTIIP.
SEQ ID NO.23 
GEDVEQSLFLSVREGDSSVINCTYTDSSSTYLYWYKQEPGAGLQLLTYIFS
NMDMKQDQRLTVLLNKKDKHLSLRIADTQTGDSAIYFCATSTFGNEKLTFGT
GTRLTIIP.
SEQ ID NO.24 
GEDVEQSLFLSVREGDSSVINCTYTDSSSTYLYWYKQEPGAGLQLLTYIFS
NMDMKQDQRLTVLLNKKDKHLSLRIADTQTGDSAIYFCATSQFGNEKLTFGT
GTRLTIIP.
SEQ ID NO.25 
GEDVEQSLFLSVREGDSSVINCTYTDSSSTYLYWYKQEPGAGLQLLTYIFS
NMDMKQDQRLTVLLNKKDKHLSLRIADTQTGDSAIYFCATSDFGNEKLTFGT
GTRLTIIP.
SEQ ID NO.26 
GEDVEQSLFLSVREGDSSVINCTYTDSSSTYLYWYKQEPGAGLQLLTYIFS
NMDMKQDQRLTVLLNKKDKHLSLRIADTQTGDSAIYFCASSQFGNEKLTFGT
 GTRLTIIP.
SEQ ID NO.27 
GEDVEQSLFLSVREGDSSVINCTYTDSSSTYLYWYKQEPGAGLQLLTYIFS
NMDMKQDQRLTVLLNKKDKHLSLRIADTQTGDSAIYFCAWSSFGNEKLTFGT
GTRLTIIP.
SEQ ID NO.28 
GEDVEQSLFLSVREGDSSVINCTYTDSSSTYLYWYKQEPGAGLQLLTYIFS
NMDMKQDQRLTVLLNKKDKHLSLRIADTQTGDSAIYFCAHSQFGNEKLTFGT
GTRLTIIP.
SEQ ID NO.29 
GEDVEQSLFLSVREGDSSVINCTYTDSSSTYLYWYKQEPGAGLQLLTYIFS
NMDMKQDQRLTVLLNKKDKHLSLRIADTQTGDSAIYFCAQSQFGNEKLTFGT
GTRLTIIP.
SEQ ID NO.30 
GEDVEQSLFLSVREGDSSVINCTYTDSSSTYLYWYKQEPGAGLQLLTYIFS
NMDMKQDQRLTVLLNKKDKHLSLRIADTQTGDSAIYFCANSTFGNEKLTFGT
GTRLTIIP.
SEQ ID NO.31 
GEDVEQSLFLSVREGDSSVINCTYTDSSSTYLYWYKQEPGAGLQLLTYIFS
NMDMKQDQRLTVLLNKKDKHLSLRIADTQTGDSAIYFCAYSQFGNEKLTFGT
GTRLTIIP.
SEQ ID NO.32

GEDVEQSLFLSVREGDSSVINCTYTDSSSTYLYWYKQEPGAGLQLLTYIFS
NMDMKQDQRLTVLLNKKDKHLSLRIADTQTGDSAIYFCAEHTHQNEKLTFGT
GTRLTIIP.

### Example 7

### Expression, renaturation, and purification of fusion body of anti-CD3 antibody and high-affinity αβ heterodimeric TCR

Fusion molecules were prepared by fusing an anti-CD3 single-chain antibody (scFv) with the αβ heterodimeric TCR. The anti-CD3 scFv was fused with the TCR β chain. The TCR β chain could contain the β chain variable domain of any of the high-affinity αβ heterodimeric TCRs described above, and the TCR α chain of the fusion molecule could contain the α chain variable domain of any of the high-affinity αβ heterodimeric TCRs described above.

### Construction of fusion molecule expression vector

(1) Construction of α chain expression vector: the target gene carrying the α chain of the αβ heterodimeric TCR was double digested by Nco I and Not I and linked to the pET28a vector which was double digested by Nco I and Not I. The linking product was transformed into E. coli DH5α, plated on a kanamycin-containing LB plate, and cultured in an inverted position at 37°C overnight. Positive clones were picked for PCR screening, and positive recombinants were sequenced. After confirming the correct sequence, recombinant plasmids were extracted and transformed into E.coli Tuner (DE3) for expression.
(2) Construction of anti-CD3 (scFv)-β chain expression vector: primers were designed to link the anti-CD3 scFv and high-affinity heterodimeric TCR β chain genes through the overlap PCR method, the short linker peptide (linker) was GGGGS, and the gene fragment of the fusion protein of the anti-CD3 scFv and high-affinity heterodimeric TCR β chain was provided with restriction endonuclease sites Nco I (CCATGG) and Not I (GCGGCCGC). The PCR amplification product was double digested by Nco I and Not I and linked to the pET28a vector which was double digested by Nco I and Not I. The linking product was transformed into E. coli DH5α competent cells, plated on a kanamycin-containing LB plate, and cultured in an inverted position at 37°C overnight. Positive clones were picked for PCR screening, and positive recombinants were sequenced. After confirming the correct sequence, recombinant plasmids were extracted and transformed into E.coli Tuner (DE3) competent cells for expression.

### Expression, renaturation, and purification of fusion protein

Expression plasmids were individually transformed into E. coli Tuner (DE3) competent cells and plated on an LB plate (kanamycin 50 ug/mL) and cultured overnight at 37°C. On the next day, the selected clones were inoculated into 10 mL of LB liquid medium (kanamycin 50 µg/mL) and cultured for 2-3 h, then inoculated into 1 L of LB medium at a volume ratio of 1:100, and further cultured to an OD₆₀₀ of 0.5-0.8. The expression of the target protein was induced by adding IPTG at a final concentration of 1 mM. Four hours after induction, cells were obtained by centrifugation at 6000 rpm for 10 min. The bacteria were washed once with a PBS buffer solution and aliquoted. The bacteria corresponding to 200 mL of bacterial culture were lysed with 5 mL of BugBuster Master Mix (Merck), and the inclusion bodies were collected by centrifugation at 6000 g for 15 min. Four detergent washings were then performed to remove cell debris and membrane components. The inclusion bodies were then washed with a buffer solution such as PBS to remove detergents and salts. Finally, the inclusion bodies were dissolved in a buffer solution containing 6 M guanidine hydrochloride, 10 mM DTT, 10 mM EDTA, and 20 mM Tris, pH8.1, and the concentration of the inclusion bodies was determined. The inclusion bodies were aliquoted and stored frozen at -80°C.

The dissolved TCR α chain and anti-CD3 (scFv)-β chain were rapidly mixed in a 2:5 mass ratio in 5 M urea, 0.4 M L-arginine, 20 mM Tris pH8.1, 3.7 mM cystamine, 6.6 mM β-mercapoethylamine (4°C) to final concentrations of 0.1 mg/mL and 0.25 mg/mL, respectively.

After mixing, the solution was placed in 10 times the volume of deionized water for dialysis (4°C). After 12 hours, the deionized water was replaced with a buffer solution (10 mM Tris, pH8.0) and continued for dialysis at 4°C for 12 hours. The dialyzed solution was filtered through a 0.45 uM filter membrane and purified through an anion-exchange column (HiTrap Q HP, 5ml, GE healthcare). The TCR with elution peaks containing the renatured successful TCR α chain and anti-CD3 (scFv)-β chain dimer was confirmed by SDS-PAGE gel. The TCR fusion molecule was then further purified by size exclusion chromatography (S-100 16/60, GE healthcare) and repurified using an anion-exchange column (HiTrap Q HP 5ml, GE healthcare). The purity of the purified TCR fusion molecule was determined by SDS-PAGE to be greater than 90%, and the concentration was determined by the BCA method.

### Example 8

### Activation function experiment of effector cells transfected with high-affinity TCR of the present application against T2 cells loaded with short peptides

IFN-γ is a potent immunoregulatory factor generated by activated T lymphocytes. Therefore, in this example, the activation function and antigen specificity of the cells transfected with the high-affinity TCR of the present application were verified by detecting the number of IFN-y through an ELISPOT experiment well known to a person skilled in the art. The high-affinity TCR of the present application (TCR numbers and their sequence numbers were known from Table 4) was transfected into CD3⁺T cells isolated from the blood of a healthy volunteer as effector cells, and CD3⁺T cells transfected with another TCR (A6) or with the wild-type TCR (WT-TCR) from the same volunteer were used as controls. The target cells used were T2 loaded with the MAGE-A4 antigen short peptide GVYDGREHTV, T2 loaded with other antigen short peptides, or unloaded T2.

ELISPOT plates were first prepared. 1×10⁴ target cells/well and 2×10³ effector cells/well (calculated according to the transfection positive rate) were first added to the corresponding wells. Then, a MAGE-A4 antigen short peptide GVYDGREHTV solution was added to the experimental group, and another antigen short peptide solution was added to the control group so that the final concentration of the short peptide was 10-6 M. Equal volume of culture medium was added to the blank group, and two duplicate wells were provided. The plates were incubated overnight (37°C, 5% CO₂). On the second day of the experiment, the plates were washed and subjected to secondary detection and color development, the plates were dried, and an immune spot plate reader (ELISPOT READER system; AID20) was used to count the spots formed on the membrane.

The experimental results were shown in Fig. 2a. With regard to the target cells loaded with the MAGE-A4 antigen short peptide GVYDGREHTV, the effector cells transfected with the high-affinity TCR of the present application had a more obvious activation effect than the effector cells transfected with the wild-type TCR, while the effector cells transfected with other TCRs were inactive. Meanwhile, the effector cells transfected with the TCR of the present application were essentially inactive against target cells loaded with other antigen short peptides or unloaded target cells.

Meanwhile, the effector cells transfected with the high-affinity TCR of the present application also had the property of binding to the GLYDGREHSV-HLA A0201 complex, as measured by the ELISOPT experiment described above. GLYDGREHSV is a short peptide derived from MAGE-A8. In the experiment, the target cells were T2 loaded with MAGE-A8 antigen short peptide GLYDGREHSV, T2 loaded with other antigen short peptides, or unloaded T2, and the effector cells were CD3⁺T cells transfected with the high-affinity TCR of the present application.

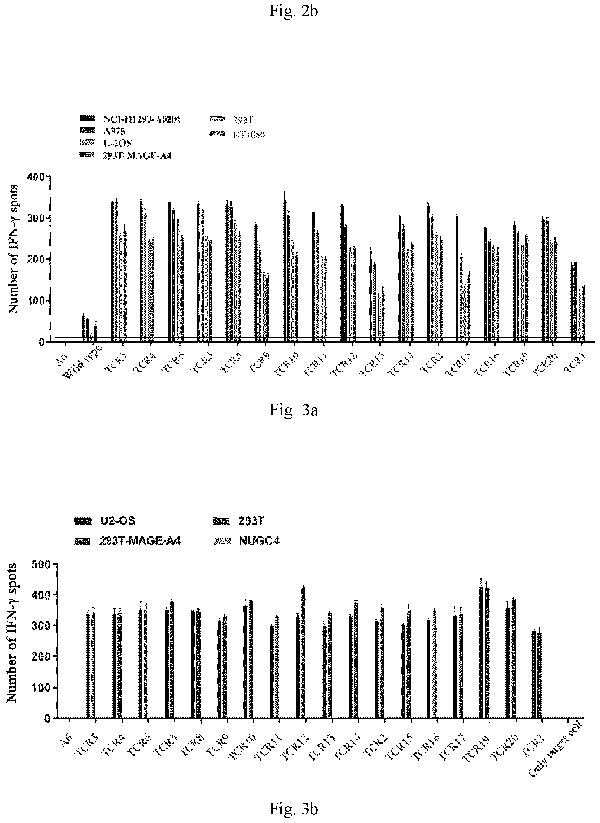

The experimental results were shown in Fig. 2b. With regard to the target cells loaded with the MAGE-A8 antigen short peptide GLYDGREHSV, the effector cells transfected with the high-affinity TCR of the present application had a more obvious activation effect than the effector cells transfected with the wild-type TCR, while the effector cells transfected with other TCRs were essentially inactive. Meanwhile, the effector cells transfected with the TCR of the present application were essentially inactive against target cells loaded with other antigen short peptides or unloaded target cells.

### Example 9

Activation function experiment of effector cells transfected with high-affinity TCR of the present application against tumor cell lines

In this example, the activation function and specificity of the effector cells transfected with the high-affinity TCR of the present application were verified again using the tumor cell lines. Detection was also performed by the ELISPOT experiment well known to a person skilled in the art. The high-affinity TCR of the present application was transfected into CD3⁺T cells isolated from the blood of a healthy volunteer as effector cells, and CD3⁺T cells transfected with another TCR (A6) or with the wild-type TCR (WT-TCR) from the same volunteer were used as controls. The tumor cell lines used in this example were NCI-H1299, A375, U-2OS, 293T, HT1080, and NUGC4. NCI-H1299 was purchased from Cell Bank of Chinese Academy of Sciences, A375, 293T, and HT1080 were purchased from ATCC, U-2OS was purchased from Guangzhou Cellcook Biotech Co., Ltd, and NUGC4 was purchased from Sciencell. The following experiments were performed in two batches (I) and (II).
(I) The high-affinity TCRs were known from Table 4 as TCR1, TCR2, TCR3, TCR4, TCR5, TCR6, TCR8, TCR9, TCR10, TCR11, TCR12, TCR13, TCR14, TCR15, TCR16, TCR19, and TCR20. The MAGE-A4 positive tumor cell lines used in this batch were NCI-H1299-A0201 (HLA-A0201 overexpression), A375 (MAGE-A4 positive), U-2OS (MAGE-A4, A8 double positive), and 293T-MAGE-A4 (MAGE-A4 overexpression), and the negative cell lines were 293T and HT1080.
(II) The high-affinity TCRs were known from Table 4 as TCR1, TCR2, TCR3, TCR4, TCR5, TCR6, TCR8, TCR9, TCR10, TCR11, TCR12, TCR13, TCR14, TCR15, TCR16, TCR17, TCR19, and TCR20. The MAGE-A4 positive tumor cell lines used in this batch were U-2OS (MAGE-A4, A8 double positive) and 293T-MAGE-A4 (MAGE-A4 overexpression), and the negative cell lines were 293T and NUGC4.

The following steps were performed. ELISPOT plates were first prepared. The ELISPOT plates were ethanol-activated and coated overnight at 4°C. On the first day of the experiment, the coating solution was removed. The ELISPOT plates were washed, blocked, and incubated at room temperature for two hours. Then, the blocking solution was removed, and the components of the experiment were added to the ELISPOT plates: 2×10⁴ target cells/well, and 2×10³ effector cells/well (calculated according to the transfection positive rate), and two duplicate wells were provided. The plates were incubated overnight (37°C, 5% CO₂). On the second day of the experiment, the plates were washed and subjected to secondary detection and color development, the plates were dried, and an immune spot plate reader (ELISPOT READER system; AID20) was used to count the spots formed on the membrane.

The experimental results were shown in Fig. 3a and Fig. 3b. With regard to the MAGE-A4 positive tumor cell line, only the target cells were inactive, and the effector cells transfected with the high-affinity TCR of the present application had a more obvious activation effect than the effector cells transfected with the wild type, while the effector cells transfected with other TCRs were inactive. Meanwhile, the effector cells transfected with the high-affinity TCR of the present application were inactive against the MAGE-A4 negative cell line.

### Example 10

### Killing function experiment of effector cells transfected with high-affinity TCR of the present application against tumor cell lines

LDH is abundant in the cytoplasm, normally cannot pass through the cell membrane, and may be released outside the cell when the cell is damaged or dead. In this case, the activity of LDH in the cell culture solution is proportional to the number of cell deaths. In this example, the killing function of the cells transfected with the TCR of the present application was also verified by measuring the release of LDH by nonradioactive cytotoxicity experiments well known to a person skilled in the art. In this example, in the LDH experiment, the high-affinity TCR of the present application was transfected into CD3⁺T cells isolated from the blood of a healthy volunteer as effector cells, and CD3⁺T cells transfected with another TCR (A6) from the same volunteer were used as controls. The tumor cell lines used in this example were U2-OS, A375, 293T, NUGC4, and MS751. U-2OS and MS751 were purchased from Guangzhou Cellcook Biotech Co., Ltd, A375 and 293T were purchased from ATCC, and NUGC4 was purchased from Sciencell. The following experiments were performed in two batches (I) and (II).
(I) The high-affinity TCRs were known from Table 4 as TCR5, TCR17, and TCR20. The MAGE-A4 positive tumor cell lines used in this batch were U-2OS (MAGE-A4, A8 double positive) and A375 (MAGE-A4 positive), and the negative tumor cell line was 293T.
(II) The high-affinity TCRs were known from Table 4 as TCR3, TCR5, and TCR19. The MAGE-A4 positive tumor cell lines used in this batch were U-2OS (MAGE-A4, A8 double positive) and 293T-MAGE-A4 (293T cells transfected with MAGE-A4), and the negative tumor cell lines were NUGC4, MS751, and 293T.

Experimental steps were as follows. LDH plates were first prepared, and the components of the experiment were added to the plates in the following order: batch (I): 3×10⁴ target cells/well and 3×10⁴ effector cells/well (calculated according to the transfection positive rate); batch (II): 2.5×10⁴ target cells/well and 7.5×10⁴ effector cells/well (calculated according to the transfection positive rate) were added into corresponding wells, and three duplicate wells were provided. Meanwhile, spontaneous wells for effector cells, spontaneous wells for target cells, maximum wells for target cells, volume correction control wells, and medium background control wells were provided. The plates were incubated overnight (37°C, 5% CO₂). On the second day of the experiment, color development was detected. After stopping the reaction, absorbance was recorded at 490 nm using a microplate reader (Bioteck).

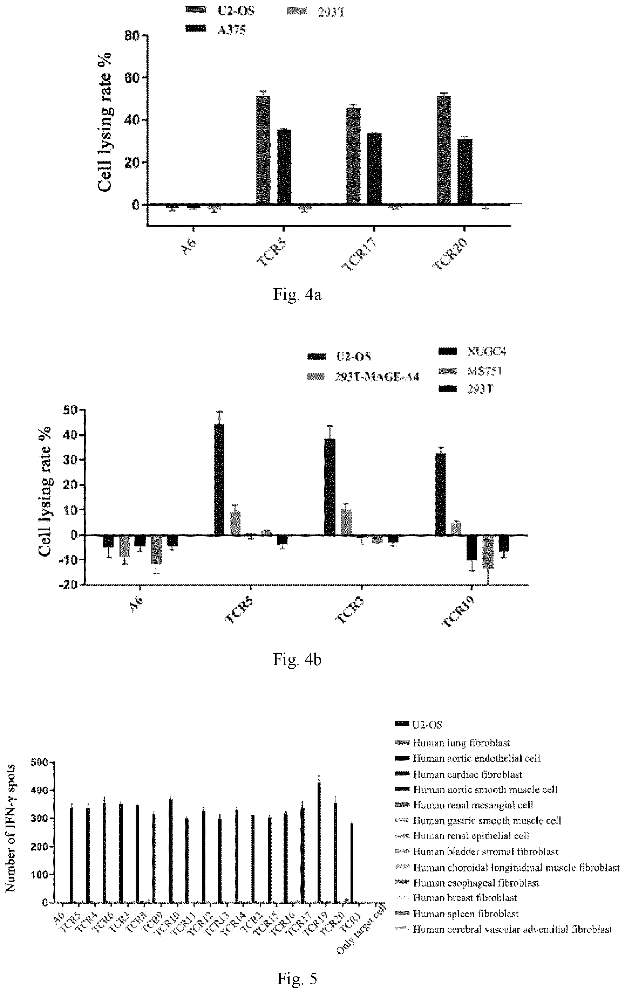

The experimental results were shown in Fig. 4a and Fig. 4b. With regard to the MAGE-A4 positive tumor cell line, the effector cells transfected with the high-affinity TCR of the present application still showed a strong killing effect, while the T cells transfected with other TCRs and the T cells not transfected with any TCR did not react. Meanwhile, the T cells transfected with the high-affinity TCR of the present application hardly killed the negative tumor cell lines.

### Example 11

### Verification of the specificity of effector cells transfected with high-affinity TCR of the present application on human normal tissue cells

In this example, the specificity of the effector cells transfected with the high-affinity TCR of the present application was verified using the human normal tissue cells. Detection was also performed by the ELISPOT experiment well known to a person skilled in the art. The high-affinity TCR of the present application (TCR numbers and their sequence numbers were known from Table 4) was transfected through lentivirus into CD3⁺T cells isolated from the blood of a healthy volunteer as effector cells, and CD3+T cells transfected with another TCR (A6) or empty-transfected (NC) from the same volunteer were used as controls.

In the experiment, the positive tumor cell line used was US-OS (MAGE-A4, A8 double positive cells), and the human normal tissue cells used were: human lung fibroblasts (HPF-a), human aortic endothelial cells (HAEC-2A), human cardiac fibroblasts (HCF-4A), human aortic smooth muscle cells (HASMC-6B), human renal mesangial cells (HRMC-11A), human gastric smooth muscle cells (HGSMC-13B), human renal epithelial cells (HREpiC-14A), human bladder stromal fibroblasts (HBdSF-39), human choroidal longitudinal muscle fibroblasts (HCPF-20), human esophageal fibroblasts (HEF-27), human breast fibroblasts (HMF-40), human spleen fibroblasts (HSF-17), and human cerebral vascular adventitial fibroblasts (HBVAF-22). The human normal tissue cells used were purchased from Sciencell.

ELISPOT plates were first prepared. The ELISPOT plates were ethanol-activated and coated overnight at 4°C. On the first day of the experiment, the coating solution was removed. The ELISPOT plates were washed, blocked, and incubated at room temperature for two hours. Then, the blocking solution was removed, and the components of the experiment were added to the ELISPOT plates: 2×10⁴ target cells/well, and 2×10³ effector cells/well (calculated according to the transfection positive rate), and two duplicate wells were provided. The plates were incubated overnight (37°C, 5% CO₂). On the second day of the experiment, the plates were washed and subjected to secondary detection and color development, the plates were dried, and an immune spot plate reader (ELISPOT READER system; AID20) was used to count the spots formed on the membrane.

As shown in Fig. 5, the effector cells transfected with the high-affinity TCR of the present application were essentially inactive against the human normal tissue cells, further verifying the high specificity of the TCR of the present application.

### Example 12

### In vivo efficacy of high-affinity TCR molecules of the present application

The T cells transfected with the high-affinity TCR of the present application were injected into mice of a human melanoma xenograft model to detect the tumor inhibitory effect in vivo.

In the experiment, NSG mice (Biocytogen) (female, 6-8 weeks old) were used as the experimental subjects. Ten days before the experiment, 1*10^7 A375 tumor cell (ATCC) suspensions were cultured, collected, suspended, and then injected subcutaneously into the one abdominal side of the mice to establish a human melanoma xenograft mouse model.

On the day of starting the experiment, a vernier caliper was used to measure the long diameter (a) and short diameter (b) of the formed tumor of each mouse, and the tumor area was calculated according to the following equation: V= (a*b^2)/2. Then, according to the groups: 5 mice (T cells transfected with irrelevant TCRs) were in the control group; 5 mice were in the T cell group transfected with TCR1 (α chain variable domain SEQ ID NO: 13, β chain variable domain SEQ ID NO:2); 5 mice were in the T cell group transfected with TCR2 (α chain variable domain SEQ ID NO:14, β chain variable domain SEQ ID NO:2); and 5 mice were in the T cell group transfected with TCR4 (α chain variable domain SEQ ID NO: 16, β chain variable domain SEQ ID NO:2). The mice were randomly grouped according to the tumor volume. After grouping, the prepared T cells were injected into the tail vein of the above mice at 1*10^7/mouse.

After the TCR-T cell injection, 200 µl of prepared IL-2 solution (50000 IU) was intraperitoneally injected into each mouse, and then the same amount of IL-2 solution was continuously injected daily for 4 days. From the beginning of the experiment, the tumor diameters were measured, and the tumor volumes were calculated once every 2 days according to the above method until the mice were affected by the oversize tumors or the tumors subsided; The above data were collated and processed for statistical analysis of tumor volume data for each group of mice.

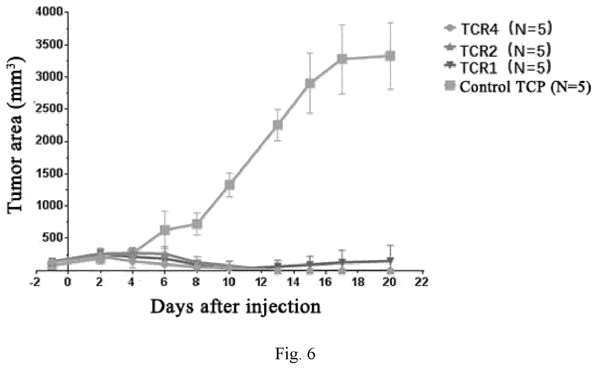

The experimental results were shown in Fig. 6. In the group of mice injected with T cells transfected with the high-affinity TCR of the present application, the growth of the tumor was significantly inhibited and showed a shrinking trend, while in the group of mice injected with T cells transfected with irrelevant TCRs, the growth of tumor volume was still fast.

The applicant states that the above are only specific implementations of the present application, and the scope of the present application is not limited thereto. A person skilled in the art should understand that any changes and substitutions which can be readily made by a person skilled in the art within the technical scope disclosed in the present application fall within the scope of protection and disclosure of the present application.

All references mentioned in the present application are incorporated herein by reference as if each reference were individually incorporated by reference. Furthermore, it is to be understood that a person skilled in the art, upon reading the above teachings of the present application, may make various changes and modifications to the present application, and these equivalent forms likewise fall within the scope limited by the claims appended to the present application.

The technical features of the above embodiments may be combined arbitrarily. In order to make the description concise, not all possible combinations of the technical features of the above-mentioned embodiments are described. However, as long as there is no contradiction between the combinations of these technical features, they should be considered to be within the scope of this specification.

## Claims

1. A T cell receptor (TCR), comprising a TCR α chain variable domain and a TCR β chain variable domain, wherein the TCR has an activity of binding to a GVYDGREHTV-HLA A0201 complex; an amino acid sequence of the TCR α chain variable domain has at least 90% sequence homology to an amino acid sequence shown in SEQ ID NO:1, and an amino acid sequence of the TCR β chain variable domain has at least 90% sequence homology to an amino acid sequence shown in SEQ ID NO:2.

2. The TCR according to claim 1, wherein three complementarity determining regions (CDRs) of the TCR β chain variable domain are: CDR1β: MNHEY, CDR2β: SVGEGT, and CDR 3β: ASSLGRAYEQY.

3. The TCR according to claim 1, wherein the amino acid sequence of the TCR β chain variable domain is SEQ ID NO:2.

4. The TCR according to claim 1, wherein the TCR further has an activity of binding to a GLYDGREHSV-HLAA0201 complex.

5. The TCR according to claim 1, wherein the amino acid sequence of the TCR α chain variable domain has at least 95% sequence homology to the amino acid sequence shown in SEQ ID NO:1, and the amino acid sequence of the TCR β chain variable domain has at least 95% sequence homology to the amino acid sequence shown in SEQ ID NO:2.

6. The TCR according to claim 1, wherein the affinity of the TCR to the GVYDGREHTV-HLAA0201 complex is at least 5 times that of a wild-type TCR.

7. The TCR according to claim 1, wherein reference sequences of three CDRs of the TCR α chain variable domain are as follows:
CDR1α: DSSSTY,
CDR2α: IFSNMDM, and
CDR3α: AEQSFGNEKLT; CDR3α contains at least one of the following mutations:
| Residue before mutation | Residue after mutation |
|---|---|
| E at position 2 of CDR3α | W, V, A, T, S, H, Q, N, or Y |
| Q at position 3 of CDR3α | H or S |
| S at position 4 of CDR3α | T, Q, N, E, or D |
| F at position 5 of CDR3α | K, R, or H |
| G at position 6 of CDR3α | Q |

8. The TCR according to claim 9, wherein an amino acid mutation in CDR3α contains:
| Residue before mutation | Residue after mutation |
|---|---|
| Q at position 3 of CDR3α | S |

9. The TCR according to claim 1, wherein the TCR has CDRs selected from the following groups:
| CDR number | α-CDR1 | α-CDR2 | α-CDR3 | β-CDR1 | β-CDR2 | β-CDR3 |
|---|---|---|---|---|---|---|
| 4 | DSSSTY | IFSNMDM | AASEFGNEKLT | MNHEY | SVGEGT | ASSLGRAYEQY |
| 2 | DSSSTY | IFSNMDM | AWSQFGNEKLT | MNHEY | SVGEGT | ASSLGRAYEQY |
| 1 | DSSSTY | IFSNMDM | AEHTKQNEKLT | MNHEY | SVGEGT | ASSLGRAYEQY |
| 3 | DSSSTY | IFSNMDM | AVSNFGNEKLT | MNHEY | SVGEGT | ASSLGRAYEQY |
| 5 | DSSSTY | IFSNMDM | AASTFGNEKLT | MNHEY | SVGEGT | ASSLGRAYEQY |
| 6 | DSSSTY | IFSNMDM | AASQFGNEKLT | MNHEY | SVGEGT | ASSLGRAYEQY |
| 7 | DSSSTY | IFSNMDM | AASQRGNEKLT | MNHEY | SVGEGT | ASSLGRAYEQY |
| 8 | DSSSTY | IFSNMDM | AVSQFGNEKLT | MNHEY | SVGEGT | ASSLGRAYEQY |
| 9 | DSSSTY | IFSNMDM | AVSDFGNEKLT | MNHEY | SVGEGT | ASSLGRAYEQY |
| 10 | DSSSTY | IFSNMDM | AVSSFGNEKLT | MNHEY | SVGEGT | ASSLGRAYEQY |
| 11 | DSSSTY | IFSNMDM | ATSTFGNEKLT | MNHEY | SVGEGT | ASSLGRAYEQY |
| 12 | DSSSTY | IFSNMDM | ATSQFGNEKLT | MNHEY | SVGEGT | ASSLGRAYEQY |
| 13 | DSSSTY | IFSNMDM | ATSDFGNEKLT | MNHEY | SVGEGT | ASSLGRAYEQY |
| 14 | DSSSTY | IFSNMDM | ASSQFGNEKLT | MNHEY | SVGEGT | ASSLGRAYEQY |
| 15 | DSSSTY | IFSNMDM | AWSSFGNEKLT | MNHEY | SVGEGT | ASSLGRAYEQY |
| 16 | DSSSTY | IFSNMDM | AHSQFGNEKLT | MNHEY | SVGEGT | ASSLGRAYEQY |
| 17 | DSSSTY | IFSNMDM | AQSQFGNEKLT | MNHEY | SVGEGT | ASSLGRAYEQY |
| 18 | DSSSTY | IFSNMDM | ANSTFGNEKLT | MNHEY | SVGEGT | ASSLGRAYEQY |
| 19 | DSSSTY | IFSNMDM | AYSQFGNEKLT | MNHEY | SVGEGT | ASSLGRAYEQY |
| 20 | DSSSTY | IFSNMDM | AEHTHQNEKLT | MNHEY | SVGEGT | ASSLGRAYEQY |

10. The TCR according to claim 1, wherein the TCR is soluble.

11. The TCR according to claim 1, wherein the TCR is an αβ heterodimeric TCR, containing an α chain TRAC constant region sequence and a β chain TRBC1 or TRBC2 constant region sequence.

12. The TCR according to claim 1, wherein the TCR contains (i) the TCR α chain variable domain and all or a part of a TCR α chain constant region except a transmembrane domain; and (ii) the TCR β chain variable domain and all or a part of a TCR β chain constant region except a transmembrane domain.

13. The TCR according to claim 1, wherein the TCR contains an α chain constant region and a β chain constant region; an artificial interchain disulfide bond is contained between the α chain constant region and the β chain constant region; preferably, cysteine residues forming the artificial interchain disulfide bond between the α chain constant region and the β chain constant region of the TCR replace one or more of the following groups of sites:
Thr48 of exon 1 of TRAC*01 and Ser57 of exon 1 of TRBC1*01 or TRBC2*01;
Thr45 of exon 1 of TRAC*01 and Ser77 of exon 1 of TRBC1*01 or TRBC2*01;
Tyr10 of exon 1 of TRAC*01 and Ser17 of exon 1 of TRBC1*01 or TRBC2*01;
Thr45 of exon 1 of TRAC*01 and Asp59 of exon 1 of TRBC1*01 or TRBC2*01;
Ser15 of exon 1 of TRAC*01 and Glu15 of exon 1 of TRBC1*01 or TRBC2*01;
Arg53 of exon 1 of TRAC*01 and Ser54 of exon 1 of TRBC1*01 or TRBC2*01;
Pro89 of exon 1 of TRAC*01 and Ala19 of exon 1 of TRBC1*01 or TRBC2*01; and
Tyr10 of exon 1 of TRAC*01 and Glu20 of exon 1 of TRBC1*01 or TRBC2*01.

14. The TCR according to claim 1, wherein the amino acid sequence of the TCR α chain variable domain is one of SEQ ID NO: 16, 14, 13, 15, or 17-32; and the amino acid sequence of the TCR β chain variable domain is SEQ ID NO:2.

15. The TCR according to claim 1, wherein the TCR is a single-chain TCR.

16. The TCR according to claim 1, wherein the TCR is a single-chain TCR comprising the α chain variable domain and the β chain variable domain; the α chain variable domain and the β chain variable domain are linked by a flexible short peptide sequence (linker).

17. The TCR according to claim 1, wherein a C- or N-terminus of the α chain and/or β chain of the TCR is bound with a conjugate;
preferably, the conjugate is a detectable marker or a therapeutic agent;
more preferably, the therapeutic agent that binds to the TCR is an anti-CD3 antibody linked to the C- or N-terminus of the α chain or β chain of the TCR.

18. A multivalent T cell receptor (TCR) complex, containing at least two TCR molecules, wherein at least one TCR molecule is the TCR according to any one of the preceding claims.

19. A nucleic acid molecule, containing a nucleic acid sequence encoding the TCR according to any one of claims 1-17 or a complementary sequence of the nucleic acid sequence.

20. A vector, containing the nucleic acid molecule according to claim 19.

21. A host cell, containing the vector according to claim 20 or having a chromosome integrated with an exogenous nucleic acid molecule according to claim 19.

22. An isolated cell, expressing the T cell receptor (TCR) according to any one of claims 1-17, wherein preferably, the isolated cell is a T cell.

23. A pharmaceutical composition, containing the T cell receptor (TCR) according to any one of claims 1-17, the TCR complex according to claim 18, or the cell according to claim 22.

24. A method for treating a disease, comprising: administering the T cell receptor (TCR) according to any one of claims 1-17, the TCR complex according to claim 18, the cell according to claim 22, or the pharmaceutical composition according to claim 23 to a subject in need of treatment, wherein preferably, the disease is a MAGE-A4 positive tumor.

25. Use of the T cell receptor (TCR) according to any one of claims 1-17, the TCR complex according to claim 18, or the cell according to claim 22 in the preparation of a medicine for the treatment of a tumor, wherein preferably, the tumor is a MAGE-A4 positive tumor.

26. A method for preparing the T cell receptor (TCR) according to any one of claims 1-17, including the steps of:
(i) culturing the cell according to claim 21 to express the TCR according to any one of claims 1-17; and
(ii) obtaining the TCR through isolating or purifying.
